# EUROPÄISCHE PATENTANMELDUNG

(11) **EP 3 061 450 A1**
(43) Veröffentlichungstag der Anmeldung: **31.08.2016**
(21) Anmeldenummer: 15156750.0
(22) Anmeldetag: 26.02.2015
(51) Int. Cl.: A61K 31/235, A61K 31/352, C07C 69/94, A61P 1/08, A61P 21/02, A61P 23/00, A61P 27/06

(54) **Mischungen cannabinoider Verbindungen, deren Herstellung und Verwendung**

(71) Anmelder: Symrise AG, 37603 Holzminden (DE)
(72) Erfinder:
(74) Vertreter: Eisenführ Speiser

(57) **Zusammenfassung**

Beschrieben werden spezifische Mischungen umfassend eine oder mehrere (cannabinoide) Verbindungen der Formel (A) und/oder ein oder mehrere von deren Salzen sowie Verfahren zu deren Herstellung.

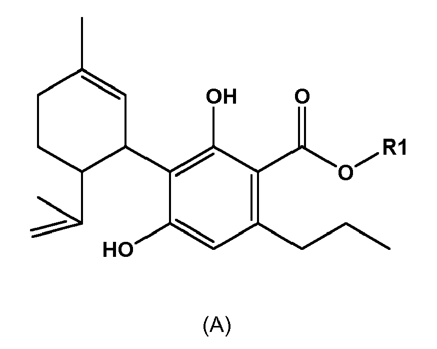

Beschrieben werden auch eine Verbindung der Formel (A), ein Salz der Formel (A) und eine entsprechende Mischung zur Anwendung als Arzneimittel bzw. zur Anwendung in einem Verfahren zur therapeutischen Behandlung des menschlichen oder tierischen Körpers.

Beschrieben werden zudem entsprechende pharmazeutische Formulierungen, kosmetische Zubereitungen und zum Verzehr geeignete, dem Genuss und/oder der Ernährung dienende Zubereitungen sowie Verfahren zur Herstellung von CBDV und THCV.

## Beschreibung

Die vorliegende Erfindung betrifft spezifische Mischungen umfassend eine oder mehrere (cannabinoide) Verbindungen der Formel (A) und/oder ein oder mehrere von deren Salzen 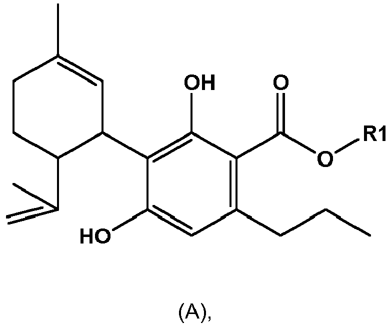 vorzugsweise eine oder mehrere (cannabinoide) Verbindungen der Formel 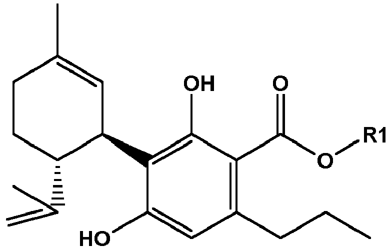 und/oder ein oder mehrere von deren Salzen (von dieser bevorzugten Verbindung),
sowie Verfahren zu deren Herstellung. Hinsichtlich der Bedeutung des Substituenten R1 siehe unten.

Für den nachfolgenden Text gilt: Ausführungsformen, die sich auf eine Verbindung der Formel (A) beziehen (und/oder deren Salze) bzw. eine solche Verbindung umfassen (und/oder deren Salze) betreffen in einer jeweils bevorzugten Ausführungsform auch Ausgestaltungen, betreffend bzw. umfassend eine Verbindung der vorstehend als bevorzugt bezeichneten (cannabinoiden) Verbindungen (und/oder deren Salze). Die Bedeutung des Substituenten R1 in der bevorzugten Verbindung ergibt sich aus der Bedeutung des Substituenten R1 für die Verbindung der Formel (A).

Die Erfindung betrifft auch eine Verbindung der obigen Formel (A), ein Salz der Formel (A) und eine Mischung umfassend eine oder mehrere (cannabinoide) Verbindungen der Formel (A) und/oder ein oder mehrere von deren Salzen, jeweils zur Anwendung als Arzneimittel bzw. zur Anwendung in einem Verfahren zur therapeutischen Behandlung des menschlichen oder tierischen Körpers.

Außerdem betrifft die vorliegende Erfindung eine Verbindung der Formel (A) bzw. ein Salz der Formel (A) bzw. eine Mischung umfassend eine oder mehrere cannabinoide Verbindungen der Formel (A) und/oder ein oder mehrere von deren Salzen zur spezifischen Anwendung in einem Verfahren zur therapeutischen Behandlung des menschlichen oder tierischen Körpers zum Erreichen einer Wirkung ausgewählt aus der Gruppe bestehend aus appetitanregende Wirkung, antiemetische Wirkung zur Hemmung von Übelkeit und Erbrechen, Reduktion muskulärer Krämpfe und Spastiken, Linderung von Schmerzsymptomen, Linderung von Migränesymptomen, Senkung des Augeninnendrucks beim Glaukom, Stimmungsaufhellung, Immunstimulation und/oder antiepileptische Wirkung.

Die vorliegende Erfindung betrifft zudem eine pharmazeutische Formulierung, umfassend eine oder mehrere Verbindungen der Formel (A) oder umfassend ein oder mehrere von deren Salzen oder umfassend eine Mischung umfassend eine oder mehrere (cannabinoide) Verbindungen der Formel (A) und/oder ein oder mehrere von deren Salzen, vorzugsweise ausgewählt aus der Gruppe bestehend aus feste galenische Formen, Dragees, Kapseln, Granulate, Pulver, Suppositorien, Lutschbonbons, Kaugummis, halbfeste Formen, Inhalate, Injektabilia, Implantate und wirkstoffhaltige Pflaster.

Weiterhin betrifft die vorliegende Erfindung kosmetische Zubereitungen und der Ernährung und/oder dem Genuss dienende, zum Verzehr geeignete Zubereitungen, umfassend eine oder mehrere Verbindungen der Formel (A) und/oder Salze davon (wie hierin beschrieben).

Die vorliegende Erfindung betrifft auch neue Verfahren zur Herstellung ausgewählter Cannabinoide.

Weiterhin betrifft die vorliegende Erfindung bestimmte, gegenüber dem Stand der Technik neue Verbindungen der Formel (A) und deren Salze (wie unten sowie insbesondere in den Patentansprüchen beschrieben).

Weitere Aspekte der vorliegenden Erfindung ergeben sich aus der nachfolgenden Beschreibung sowie den beigefügten Patentansprüchen.

Seit der Entdeckung des endogenen Cannabinoid-Systems mit seiner funktionellen Bedeutung für die Regulation und Modulation des Immun- sowie Nervensystems besteht ein ständiger Bedarf an natürlichen und künstlichen Cannabinoiden zu dessen selektiver pharmazeutischen Steuerung. Insbesondere besteht Bedarf, die Cannabinoid-Rezeptoren CB1, welche vor allem in Nervenzellen, in höchster Dichte in den Basalganglien, im Hippocampus und im Cerebellum, zu finden sind, und die Cannabinoid-Rezeptoren CB2, welche vorwiegend auf Zellen des Immunsystems und auf am Knochenaufbau und -abbau beteiligten Zellen zu finden sind, aufgrund ihrer unterschiedlichen medizinischen Funktionen gezielt separat zu stimulieren.

Die Cannabinoid-Rezeptoren CB1 und CB2 gelten als akzeptierte Wirkorte für Moleküle mit cannabinoider Struktur. Obwohl noch weitere Rezeptoren als potentieller CB3 Rezeptor diskutiert werden, wird davon ausgegangen, dass die Hauptwirkungen durch CB1 und CB2 vermittelt werden. Delta-9-THC, endogene Cannabinoide und eine Vielzahl synthetischer Cannabinoide kuppeln an die genannten Rezeptoren und üben über sie Effekte auf die Zellen aus (Pertwee, R. G. et al. Pharmacol. Rev. 2010, 62, 588-631).

CB1 und CB2 sind Mitglieder der Superfamilie der G-Protein-gekoppelten Rezeptoren (GPCRs, G Protein Coupled Receptors). Genauer gesagt hemmen die Rezeptoren über das heteromere G-Protein die Andenylatcyclase und aktivieren die mitogen aktivierte Protein-Kinase (Howlett, A. C. et al. Pharmacol. Rev. 2002, 54, 161-202; Howlett, A. C. Handb. Exp. Pharmacol. 2005, 168, 53-79). Für den CB1-Rezeptor ist weiterhin beschrieben, dass er über Ionen-Kanäle des A-Typs Kalium Ströme und über N sowie P/Q-Typ Kanäle Calcium Ströme modulieren kann. Weiterhin können CB1-Rezeptoren über Gₛ-Proteine Signale an die exprimierenden Zellen übertragen (Glass, M.,Felder, C. C. J. Neurosci. 1997; 17, 5327-5333; Maneuf, Y. P., Brotchie, J. M. J. Pharmacol. 1997; 120, 1397-1398; Calandra, B. et al. Eur. J. Pharmacol. 1999; 374, 445-455; Jarrahian, A. et al. J. Pharmacol. Exp. Ther. 2004, 308, 880-886).

Die Fähigkeit von CB1 und CB2 über G_{i/o} und im weiteren Downstream über die Hemmung der Adenylatcyclase Signale zu vermitteln, wird im sogenannten [³⁵S]GTP gammaS binding assay und dem cAMP assay (Howlett, A. C. et al. Pharmacol. Rev. 2002, 54, 161-202; Pertwee, R. G. Handb. Exp. Pharmacol. 2005a, 168, 1-51) verwendet, um die Bindung und die Signalübertragung von Cannabinoiden zu untersuchen.

CB1-Rezeptoren verfügen sowohl über eine orthosterische als auch über eine oder mehrere allosterische Bindungsstellen, die als potentielle Wirkorte für Liganden in Frage kommen (Price, M. R. et al. Mol. Pharmacol. 2005a, 68, 1484-1495; Adam, L. et al. 17th Annual Symposium of the Cannabinoids, 2007, S. 86; Horswill, J. G. et al. J. Pharmacol. 2007, 152, 805-814; Navarro, H. A. et al. J. Pharmacol. 2009, 156, 1178-1184). CB1-Rezeptoren werden hauptsächlich an den terminalen Enden von zentralen und peripheren Nervenzellen gefunden, wo sie üblicherweise eine Hemmung von exzitatorischen und inhibitorischen Neurotransmittern vermitteln (Howlett, A. C. et al. Pharmacol. Rev. 2002, 54, 161-202; Pertwee, R. G., Ross, R. A. Prostaglandins Leukot Essent Fatty Acids, 2002, 66, 101-121; Szabo, B., Schlicker, E. Handb. Exp. Pharmacol. 2005, 168, 327-365). Die Verteilung dieser Rezeptoren im zentralen Nervensystem ist dergestalt, dass ihre Aktivierung verschiedene kognitive Prozesse beeinflussen können (z.B. die Aufmerksamkeit und das Gedächtnis, verschiedene motorische Funktionen und die Schmerzwahrnehmung).

CB2-Rezeptoren sind vornehmlich wie bereits erwähnt in Immunzellen lokalisiert. Werden sie aktiviert, modulieren Sie die Zellmigration und die Ausschüttung von Cytokinen innerhalb und außerhalb des Gehirns (Howlett, A. C. et al. Pharmacol. Rev. 2002, 54, 161-202; Cabral, G. A., Staab, A. Handb. Exp. Pharmacol. 2005, 168, 385-423; Pertwee, R. G. Handb. Exp. Pharmacol. 2005a, 168, 1-51).

Es gibt außerdem Nachweise dafür, dass erstens CB1-Rezeptoren von nicht-neuronalen Zellen (einschließlich Immunzellen) (Howlett, A. C. et al. Pharmacol. Rev. 2002, 54, 161-202) und dass zweitens CB2-Rezeptoren von manchen Zellen innerhalb und außerhalb des Gehirns exprimiert werden (Skaper, S. D. et al. Proc. Natl. Acad. Sci. USA 1996, 93, 3984-3989; Ross, R. A. et al. Neuropharmacology 2001a, 40, 221-232; Van Sickle, M. D. et al. Science 2005, 310, 329-332; Wotherspoon, G. et al. Neuroscience 2005, 135, 235-245; Beltramo, M. et al. Eur. J. Neurosci. 2006, 23, 1530-1538; Gong, J. P. et al. Brain Res. 2006, 1071, 10-23; Baek, J. H. et al. Acta Otolaryngol 2008, 128, 961-967).

Bekannte Verbindungen, die nachgewiesenermaßen eine Affinität zu den oben genannten Rezeptoren CB1 und CB2 aufweisen, sind das u.a. aus den Vertretern des weiblichen Hanfs *Cannabis sativa* und *Cannabis indica* stammende Cannabidiol (CBD) sowie bestimmte chemische Derivate davon.

Cannabis gehört zur Familie der Cannabidaceae. Die botanische und chemotaxonomische Einteilung der Gattung Cannabis geschieht anhand zweier unterschiedlicher Vorgehensweisen. Schultes et al. unterscheidet drei Arten Cannabis sativa Linnaeus, Cannabis indica LAM. und Cannabis ruderalis (Schultes, R. E. et al. Harvard University Botanical Museum Leaflets 1974, 23, 337-367). Andere benennen nur die eine Sammelart Cannabis sativa L. aus den Unterarten Cannabis sativa ssp. sativa und ssp. indica.

Nach der fachjuristischen Sichtweise wird in einen Drogen- und einen Fasertyp unterschieden, wobei die Differenzierung aufgrund des mengenmäßigen Verhältnisses der Hauptcannabinoide CBD und Delta-9-THC geschieht.

Aus dem Stand der Technik sind verschiedene cannabinoide Verbindungen und Verfahren zu deren Herstellung bekannt.

Korte et al. (Tetrahedron Letters 1969, 3, 145-7) beschreiben Cannabidivarin erstmalig und schlagen eine Synthese analog der von Petrzilka et al. (Helvetica Chimica Acta 1967, 50, 719-723) vor. Hiermit lassen sich jedoch nur geringe Ausbeuten erzielen.

Auch Crombie et al. (Phytochemistry 1975, 4, 11975) beschreiben die Synthese von Cannabidivarin im Miniaturmaßstab, als Kondensation von Divarin mit para-Menthadienol. Die Synthese in getrocknetem CH₂Cl₂, gesättigt mit PTSA, ist jedoch wenig selektiv und die zu erwartenden Produkte entstehen in einem unwirtschaftlichen Verhältnis. Tetrahydrocannabidivarin (hier als Delta-1-Tetrahydrocannabivarol bezeichnet) entsteht auf demselben Weg bei höherer Temperatur in unwirtschaftlicher Konzentration in einem Vielstoffgemisch.

Ausgehend davon sind mehrere Verfahren zur Herstellung von Cannabinoiden entwickelt worden.

So beschreibt WO 2006/136273 ein Verfahren zur Herstellung von Dronabinol (im WO-Dokument bezeichnet als: (6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹-THC)), heute auch gemäß IUPAC bezeichnet als (6a*R*, 10a*R*)-6,6,9-Trimethyl-3-pentyl-6a,7,8,10a-tetrahydro-6*H-*benzo[c]chromen-1-ol oder bezeichnet als Delta-9-Tetrahydrocannabinol, Delta-9-THC oder Δ-9-THC) durch Cyclisierung von Cannabidiol (CBD) (2-[1R-3-Methyl-6-(1-methylethenyl)-2-cyclohexen-1-yl]-5-pentyl-1,3-benzoldiol) zu Delta-9-THC. Das beschriebene Verfahren ist dadurch gekennzeichnet, dass Cannabidiol (CBD) in einem organischen Lösungsmittel vorgelegt und in Gegenwart eines Molekularsiebs unter Erhitzen zu Delta-9-THC cyclisiert wird. Es wird in der WO 2006/136273 festgestellt, dass das eingesetzte Molekularsieb, neben den bisher beschriebenen trocknenden Eigenschaften, auch starke katalytische Eigenschaften besitzt, die bei der beschriebenen Umsetzung im Vordergrund stehen. Cyclisierungen, die nur in Gegenwart eines Lewis-Säure Katalysators ausgeführt werden, sind in der Regel deutlich langsamer und liefern schlechtere Ausbeuten an Delta-9-THC, als Cyclisierungen, die in Gegenwart eines Molekularsiebes durchgeführt werden.

In der Literatur sind weitere Synthesevarianten beschrieben wie z.B. von Crombie et al. (Chem. Research 1977, 114, 1301-1345). Neuere Syntheseverfahren werden u.a. in EP 23 14 580 offenbart. Das dort beschriebene Verfahren zur Herstellung von Cannbinoiden soll anwendbar sein für alle Stereoisomeren und Homologen von Cannabinoiden und besteht aus zwei bzw. drei chemischen Syntheseschritten. Dabei werden in einem ersten Schritt Alkylresorcylsäureester (6-Alkyl-2,4-dihydroxybenzoesäureester) mit ungesättigten Kohlenwasserstoffen, Alkoholen, Ketonen (bzw. deren Derivaten wie Enolestern, Enolethern und Ketalen) zu den entsprechenden in 3-Stellung substituierten 6-Alkyl-2,4-dihydroxybenzosäureestern kondensiert. Im Weiteren beschreibt die EP 25 78 561 ebenfalls die Umsetzung von Menthadienol oder dessen Derivate mit 6-Alkyl-2,4-dihydroxy-benzoesäureestern. Als bevorzugte Lewis-Säure-Katalysatoren werden sogenannte Metall-Triflate verwendet. Hierbei handelt es sich z. B. um Scandium- bzw. Zn-trifluorsulfonat. In einem zweiten Schritt werden in den beiden Veröffentlichungen die im ersten Schritt erzeugten Zwischenprodukte mit Esterfunktion einer decarboxylierenden Verseifung unterzogen, wodurch die entsprechenden esterfreien Cannabinoide entstehen. Sofern nötig wird in einem dritten Schritt eine sauer katalysierte Umlagerung vorgenommen. Diese Isomerisierung kann z. B. der Ringschluss des Pyranrings bei CBDV zu THCV sein.

US 5,342,971 beschreibt ein Verfahren zur Herstellung von Dronabinol und verwandten Dibenzo[b,d]pyranen. Diese werden gemäß des Abstracts durch Erhitzen eines Dihydroxybenzoesäurederivates in Gegenwart eines Lewis-Säure Katalysators und eines inerten unpolaren Lösungsmittels, in welchem zwar die Dihydroxybenzoesäure löslich, der Lewis-Säure Katalysator jedoch unlöslich oder sehr geringfügig löslich ist, hergestellt. Eine typische Ausgestaltung beinhaltet die Herstellung von für die Synthese von Dronabinol und verwandten Dibenzo[b,d]pyranen nützlichen Intermediaten.

Allerdings ist keine weitere Synthese von cannabinoiden Verbindungen bekannt, die zu einer 5-Propylbenzen-Struktur bzw. einem 3-propyl substituiertem Benzochromen-Grundgerüst führen.

Primäre Aufgabe der vorliegenden Erfindung war es, cannabinoid wirksame Stoffe oder Stoffgemische (und Verfahren zu deren Herstellung) anzugeben, vorzugsweise solche, die eine vorteilhafte CB1- bzw. CB2-Affinität aufweisen, wobei besonders bevorzugt eine der beiden genannten Rezeptoraffinitäten gegenüber der anderen überwiegt. Das Herstellungsverfahren sollte vorzugsweise eine gute Raum-Zeit-Ausbeute in Verbindung mit ökologischen Vorteilen (bevorzugt Verwendung nicht-chlorhaltiger Lösungsmittel) besitzen. Insbesondere sollte ein neues Verfahren zur Herstellung von CBDV, vorzugsweise aus vormals unbekannten Cannabinoid-Estern, sowie von THCV gefunden werden.

Die anzugebenden Stoffe oder Stoffgemische sollten dabei vorzugsweise als Arzneimittel oder in einem Verfahren zur therapeutischen Behandlung des menschlichen oder tierischen Körpers zum Erreichen einer Wirkung ausgewählt aus der Gruppe bestehend aus appetitanregende Wirkung, antiemetische Wirkung zur Hemmung von Übelkeit und Erbrechen, Reduktion muskulärer Krämpfe und Spastiken, Linderung von Schmerzsymptomen, Linderung von Migränesymptomen, Senkung des Augeninnendrucks beim Glaukom, Stimmungsaufhellung, Immunstimulation und/oder antiepileptischen Wirkung angewendet werden können.

Die vorliegende Erfindung basiert u.a. auf der überraschenden Erkenntnis, dass Verbindungen der Formel (A) sowie deren Salze, wobei der Substituent R1 in der Formel (A) ein aliphatischer Rest mit keiner oder einer, zwei, drei oder mehr als drei Hydroxygruppen ist, wobei die Gesamtzahl der C-Atome in dem aliphatischen Rest R1 nicht größer ist als 15, vorzugsweise nicht größer ist als 12, und
wobei der aliphatische Rest
- gesättigt oder ungesättigt ist
und
- verzweigt oder unverzweigt ist,
und
- acyclisch oder cyclisch ist,
mit der Maßgabe, dass die Verbindung(en) der Formel (A) bzw. das/die Salz(e) davon für den Fall, dass R1 ein aliphatischer Rest mit keiner Hydroxygruppe ist, ausgewählt ist/sind aus der Gruppe bestehend aus den Verbindungen Cyclohexylcannabidivarinolat (CHCBDV) und Hexylcannabidivarinolat (HCBDV) sowie deren Salzen,
eine vorteilhafte Bindungsaffinität gegenüber den Cannabinoid-Rezeptoren CB1 und CB2 aufweisen, wodurch sie sich zur Anwendung als Arzneimittel oder zur Anwendung in einem Verfahren zur therapeutischen Behandlung des menschlichen oder tierischen Körpers eignen.

Die vorstehend und ggf. nachfolgend genannten vorteilhaften bzw. überraschenden Erkenntnisse bzw. Eigenschaften gelten für die in Absatz 1 des vorliegenden Textes offenbarte bevorzugte Verbindung entsprechend.

Die Erfindung betrifft somit auch eine Verbindung der Formel (A) (wie vorstehend und nachfolgend, insbesondere in den Ansprüchen definiert) oder ein Salz einer Verbindung der Formel (A) (wie vorstehend und nachfolgend, insbesondere in den Ansprüchen definiert) oder einer Mischung (wie vorstehend und nachfolgend, insbesondere in den Ansprüchen definiert)
(i) zur Anwendung als Arzneimittel
   oder
(ii) zur Anwendung in einem Verfahren zur therapeutischen Behandlung des menschlichen oder tierischen Körpers. Die vorstehend genannte Anwendung als Arzneimittel oder Anwendung in einem Verfahren zur therapeutischen Behandlung des menschlichen oder tierischen Körpers (sowie nachfolgend genannten Anwendungen dieser Art bzw. spezifische Anwendungen als Arzneimittel oder zur Anwendung in einem spezifischen Verfahren zur therapeutischen Behandlung des menschlichen oder tierischen Körpers) gilt besonders bevorzugt für die in Absatz 1 dieses Textes offenbarte bevorzugte Verbindung.

Die Anwendung einer oder mehrerer Verbindungen der Formel (A) (wie vorstehend und nachfolgend, insbesondere in den Ansprüchen definiert) oder eines oder mehrerer von deren Salzen oder einer entsprechenden Mischung (wie vorstehend und nachfolgend, insbesondere in den Ansprüchen definiert) als Arzneimittel bzw. in einem Verfahren zur therapeutischen Behandlung des menschlichen oder tierischen Körpers zielt dabei insbesondere auf das Erreichen einer Wirkung ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
und/oder
- antiepileptische Wirkung
ab.

Die Erfindung betrifft zudem eine pharmazeutische Formulierung, umfassend eine oder mehrere Verbindungen der Formel (A) (wie vorstehend und nachfolgend, insbesondere in den Ansprüchen definiert) oder umfassend ein oder mehrere von deren Salzen (wie vorstehend und nachfolgend, insbesondere in den Ansprüchen definiert) oder umfassend eine entsprechende Mischung (wie vorstehend und nachfolgend, insbesondere in den Ansprüchen definiert). Die erfindungsgemäße pharmazeutische Formulierung ist dabei vorzugsweise ausgewählt aus der Gruppe bestehend aus
- feste galenische Formen,
- Dragees,
- Kapseln,
- Granulate,
- Pulver,
- Suppositorien,
- Lutschbonbons,
- Kaugummis,
- halbfeste Formen,
- Inhalate,
- Injektabilia
- Implantate
und
- wirkstoffhaltige Pflaster.

Alternativ liegt die pharmazeutische Formulierung in flüssiger Form vor.

Bevorzugte pharmazeutische Formulierungen sind:
Feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne), Granulate (mit modifizierter Freisetzung und ohne), Pulver (mit modifizierter Freisetzung und ohne, z.B. Nasenpulver, Ohrenpuder), Suppositorien (mit Überzug und ohne, mit modifizierter Freisetzung und ohne), Lutschbonbons, Kaugummis, halbfeste Formen (wie z.B. hydrophobe Salben darunter z.B.: Kohlenwasserstoffgele, Lipogele, Silikongele, Oleogele sowie wasseraufnehmende Salben darunter z.B. Absorptionsbasen, hydrophile Salben, hydrophile Gele (Hydrogele) oder Pasten, auch Nasensalben), Inhalate (wie z.B. Druckgasdosierinhalatoren, Pulver-Inhalatoren, Inhalatoren mit Zerstäuber, Inhalationskonzentrate zur Inhalation), Injektabilia und Implantate (z.B. auf Basis flüssiger Formen oder fester Formen, die zur Zubereitung von oder selbst als injektionsfähigen Lösungen geeignet sind, oder feste Matrizen, die eine modifizierte Freisetzung möglich machen), wirkstoffhaltige Pflaster, Ohrentampons.

Flüssige Formen sind z.B. Lösungen, Suspensionen, Emulsionen, Sirupe (umgangssprachlich Hustensaft), Mundspülungen, Gurgellösungen, Halssprays oder Nasensprays, Nasentropfen, Nasenspüllösungen, Ohrentropfen, Ohrensprays und Ohrenspüllösungen.

Pharmazeutische Formulierungen umfassend eine oder mehrere Verbindungen der Formel (A) (wie vorstehend und nachfolgend, insbesondere in den Ansprüchen definiert) und/oder ein oder mehrere von deren Salzen (wie vorstehend und nachfolgend, insbesondere in den Ansprüchen definiert) und/oder entsprechende Mischungen (wie vorstehend und nachfolgend, insbesondere in den Ansprüchen definiert) zur Anwendung als Arzneimittel oder zur Anwendung in einem Verfahren zur therapeutischen Behandlung des menschlichen oder tierischen Körpers enthalten vorzugsweise eine oder mehrere Bestandteile aus den folgenden Gruppen: Füllstoffe (z.B. Cellulose, Calciumcarbonat), Fließ- und Rieselmittel (z.B. Talkum, Magnesiumstearat), Überzüge (z.B. Polyvinylacetatphtalat, Hydroxypropylmethylcellulosephtalat), Sprengmittel (z.B. Stärke, quervernetztes Polyvinylpyrrolidon), Weichmacher (z.B. Triethylcitrat, Dibutylphtalat) Stoffe zur Granulierung (Lactose, Gelatine), Retardierung (z.B. Poly(meth)acrylsäure-methyl/ethyl/2-trimethyl-aminoethylester-Copolymerisate in Dispersion, Vinylacetat/ Crotonsäure-Copolymerisate), Kompaktierung (z.B. Mikrokristalline Cellulose, Lactose), Lösungs-, Suspendier- oder Dispergiermittel (z.B. Wasser, Ethanol), Emulgatoren (z.B. Cetylalkohol, Lecithin), Stoffe zur Veränderung der rheologischen Eigenschaften (Siliciumdioxid, Natriumalginat), Stoffe zur mikrobiellen Stabilisierung (z.B. Benzalkoniumchlorid, Kaliumsorbat), Konservierungsmittel und Antioxidantien (z.B. DLalpha-Tocopherol, Ascorbinsäure), Stoffe zur Veränderung des pH-Wertes (Milchsäure, Citronensäure), Treib- oder Inert-Gase (z.B. Fluorierte Chlorkohlenwasserstoffe, Kohlendioxid), Farbstoffe (Eisenoxide, Titandioxid), Salbengrundstoffe (z.B. Paraffine, Bienenwachs), u.a. wie sie in der Fachliteratur (z.B. Schmidt, P. C., Christin, I. "Wirk- und Hilfsstoffe für Rezeptur, Defektur und Großherstellung", 1999, Wissenschaftliche Verlagsgesellschaft mbH Stuttgart oder Bauer, K. H.,, Frömming, K-H., Führer, C. "Lehrbuch der Pharmazeutischen Technologie", 8. Auflage, 2006, Wissenschaftliche Verlagsgesellschaft mbH Stuttgart) vorkommen.

Die in einer pharmazeutischen Formulierung vorzugsweise einzusetzenden Mengen von einer oder mehreren Verbindungen der Formel (A) (wie vorstehend und nachfolgend, insbesondere in den Ansprüchen definiert) und/oder von einem oder mehreren von deren Salzen (wie vorstehend und nachfolgend, insbesondere in den Ansprüchen definiert) und/oder von entsprechenden Mischungen (wie vorstehend und nachfolgend, insbesondere in den Ansprüchen definiert) sowie von oben aufgeführten Bestandteilen können in Abhängigkeit von Art und Zweck der jeweiligen Formulierung vom Fachmann durch einfaches Ausprobieren leicht ermittelt werden.

Das vorstehend zu pharmazeutischen Formulierungen Gesagte gilt für die in Absatz 1 offenbarte bevorzugte Verbindung entsprechend.

Die hierin beschriebenen Verbindungen der Formel (A) und deren Salze eignen sich vorteilhafterweise auch zur Verwendung in kosmetischen Zubereitungen. Fernen sind sie dazu geeignet, in dem Genuss und/oder der Ernährung dienenden, zum Verzehr geeigneten Zubereitungen eingesetzt zu werden. Die in solchen Zubereitungen vorzugsweise einzusetzenden Mengen von einer oder mehreren Verbindungen der Formel (A) (wie vorstehend und nachfolgend, insbesondere in den Ansprüchen definiert) und/oder von einem oder mehreren von deren Salzen (wie vorstehend und nachfolgend, insbesondere in den Ansprüchen definiert) und/oder von entsprechenden Mischungen (wie vorstehend und nachfolgend, insbesondere in den Ansprüchen definiert) können in Abhängigkeit von Art und Zweck der jeweiligen Zubereitung vom Fachmann durch einfaches Ausprobieren leicht ermittelt werden. Bei den übrigen Bestandteilen dieser Zubereitungen handelt es sich um für solche Zubereitungen ansonsten übliche Bestandteile.

Die in einer erfindungsgemäßen Formulierung bzw. Zubereitung enthaltene Menge an Verbindung(en) der Formel (A) und/oder Salz(en) davon ist vorzugsweise ausreichend, um bei der Anwendung bzw. bei Gebrauch oder Verzehr eine oder mehrere Wirkung(en) ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
und
- antiepileptische Wirkung
zu erreichen.

Die vorliegende Erfindung betrifft auch eine Mischung umfassend eine oder mehrere Verbindungen der Formel (A) und/oder ein oder mehrere von deren Salzen, 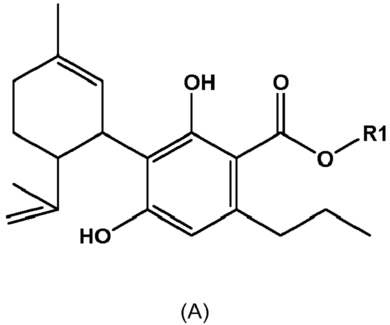 vorzugsweise eine oder mehrere (cannabinoide) Verbindungen der Formel 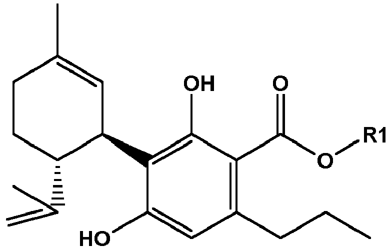 und/oder ein oder mehrere von deren Salzen,
wobei R1 ein aliphatischer Rest mit keiner oder einer, zwei, drei oder mehr als drei Hydroxygruppen ist, wobei die Gesamtzahl der C-Atome in dem aliphatischen Rest R1 nicht größer ist als 15, vorzugsweise nicht größer als 12,
und wobei der aliphatische Rest
- gesättigt oder ungesättigt ist
und
- verzweigt oder unverzweigt ist
und
- acyclisch oder cyclisch ist,
mit der Maßgabe, dass die Verbindung(en) der Formel (A) bzw. das/die Salz(e) davon für den Fall, dass R1 ein aliphatischer Rest mit keiner Hydroxygruppe ist, ausgewählt ist/sind aus der Gruppe bestehend aus den Verbindungen Cyclohexylcannabidivarinolat und Hexylcannabidivarinolat sowie deren Salzen,
wobei in der Mischung
das molare Verhältnis der Gesamtmenge an Verbindungen der Formel (A) und deren Salzen zur Menge an Verbindung der Formel (V) (Cannabidivarin, CBDV) (sofern vorhanden) 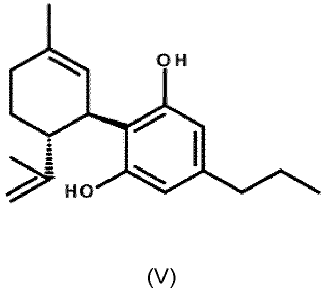 größer ist als 1 : 1, bevorzugt größer ist als 5 : 1, besonders bevorzugt größer ist als 10 : 1, und gleichzeitig
das molare Verhältnis der Gesamtmenge an Verbindungen der Formel (A) und deren Salzen zur Menge an der Verbindung der Formel (III) ((-)-trans-Methylcannabidivarinolat) (sofern vorhanden) 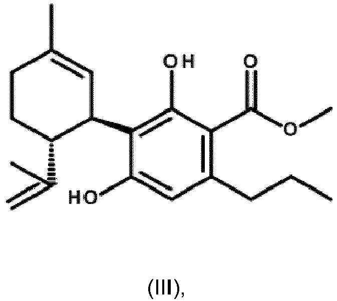 vorzugsweise zur Menge an Verbindung(en) der Formel (IX) (s. unten) (sofern vorhanden), größer ist als 1 : 1, bevorzugt größer ist als 5 : 1, besonders bevorzugt größer ist als 10 : 1.

Sofern der aliphatische Rest R1 einer Verbindung der Formel (A) ein oder mehrere chirale Zentren besitzt, ist jede der möglichen Konfigurationen an dem bzw. jedem dieser Zentren gleichwertig (R oder S). Sofern im Einzelfall nicht anders angegeben, bezeichnet eine im vorliegenden Text zeichnerisch dargestellte individuelle Verbindung der Formel (A) mit einem oder mehreren chiralen Zentren im aliphatischen Rest sämtliche Konfigurationsisomere und sämtliche Mischungen von Konfigurationsisomeren der dargestellten Verbindung gleichermaßen, sofern diese durch Einstellung der Konfiguration an dem oder den chiralen Zentren des aliphatischen Restes darstellbar sind.

Die vorliegende Erfindung (in seinen vorstehend und nachfolgend genannten, vorzugsweise als bevorzugt definierten Ausführungsformen) bezieht sich somit auch auf die folgenden bevorzugten spezifischen Verbindungen (und/oder deren Salzen) (zur Bedeutung des Substituenten R1 siehe die Bedeutung von R1 in der Verbindung der Formel (A): 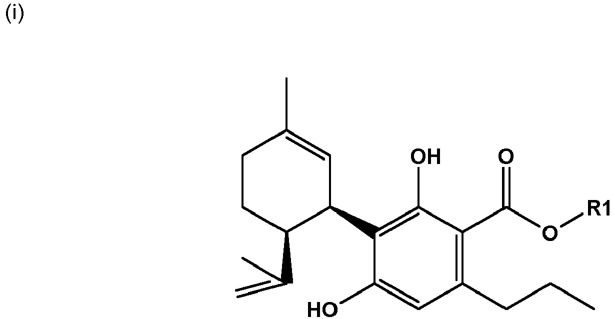 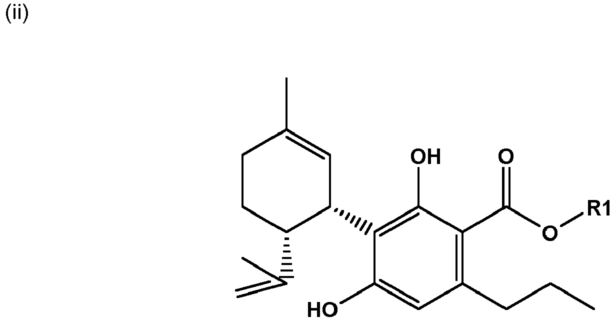 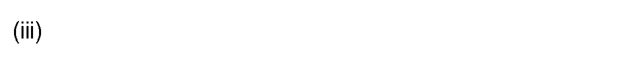 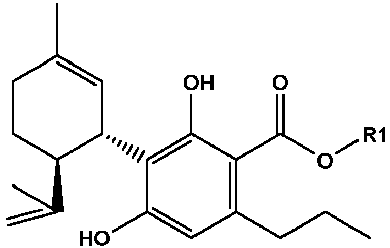 und die besonders bevorzugte Verbindung (und/oder deren Salze) der Formel

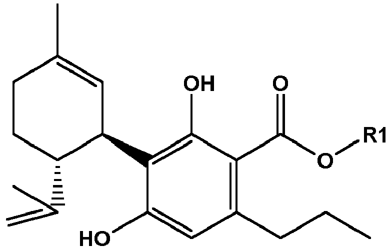

Je nach gewünschter Ausgestaltung und Zweck können auch erfindungsgemäße Mischungen (wie vorstehend und nachfolgend, insbesondere in den Ansprüchen definiert) einen oder mehrere der vorangehend im Zusammenhang mit erfindungsgemäßen pharmazeutischen Formulierungen genannten Bestandteile enthalten. Mischungen im Sinne der vorliegenden Erfindung können auch Halbfertigwaren zur Herstellung weiterer Verbindungen der Gruppe der Cannabinoide sein, die ihrerseits wiederum der Herstellung von pharmazeutischen Formulierungen dienen.

Aus einer Verbindung der Formel (A) lässt sich durch decarboxylierende Verseifung analog EP 2 314 580 A1 CBDV (Verbindung der Formel (V)) herstellen.

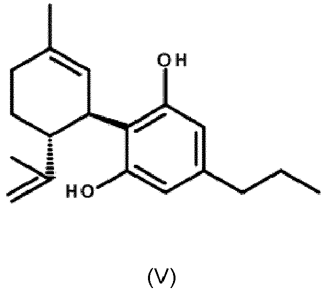

In einer erfindungsgemäßen Mischung überwiegt die Gesamtmenge an Verbindungen der Formel (A) und deren Salzen im Vergleich mit gegebenenfalls vorhandenem CBDV.

Die Verbindung(en) der Formel (A) lassen sich durch Umesterung von Verbindungen der Formel (IX) (s. unten), beispielsweise durch Umesterung des (-)-trans-Methylcannabidivarinolats der Formel (III) herstellen.

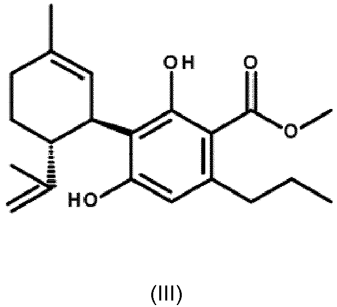

In einer erfindungsgemäßen Mischung überwiegt jedoch die Gesamtmenge an Verbindungen der Formel (A) und deren Salzen im Vergleich mit gegebenenfalls vorhandener Verbindung der Formel (III), vorzugsweise im Vergleich mit gegebenenfalls vorhandenen Verbindungen der Formel (IX) (s. unten).

In erfindungsgemäßen Mischungen können demnach beispielsweise CBDV und/oder (-)-trans-Methylcannabidivarinolat vorhanden sein, ihre Anwesenheit ist jedoch nicht verpflichtend.

Soweit eine erfindungsgemäße Mischung lediglich eine einzelne Verbindung der Formel (A) bzw. ein einzelnes Salz dieser einzelnen Verbindung der Formel (A) umfasst, enthält sie zumindest einen weiteren Bestandteil. Zu bevorzugten Bestandteilen siehe oben.

Eine erfindungsgemäße Mischung umfasst demnach beispielsweise (i) eine einzelne Verbindung oder (ii) ein einzelnes Salz oder (iii) mehrere Verbindungen oder (iv) mehrere Salze oder (v) eine Verbindung und ein Salz oder (vi) mehrere Verbindungen und ein oder mehrere Salze oder (vii) unterschiedliche Salze der gleichen Verbindung mit gleichem Deprotonierungsmuster (jedoch mit unterschiedlichen Kationen) oder (viii) im Deprotonierungsgrad sich unterscheidende Salze der gleichen Verbindung mit gleichen Kationen oder (ix) im Deprotonierungsgrad sich unterscheidende Salze der gleichen Verbindung jedoch mit gleicher oder unterschiedlichen Kationen oder (x) Salze unterschiedlicher Verbindungen mit gleichem Deprotonoierungsmuster und gleichen Kationen oder (xi) Salze unterschiedlicher Verbindungen mit unterschiedlichen Deprotonierungsmustern und gleichen oder unterschiedlichen Kationen oder (xii) Salze unterschiedlicher Verbindungen mit unterschiedlichem Deprotonierungsmuster und unterschiedlichen Kationen.

Eine erfindungsgemäße Mischung ist vorzugsweise so zusammengesetzt, dass der Anteil der Gesamtmenge an Verbindungen der Formel (A) und deren Salzen in der Mischung bezogen auf das Gesamtgewicht der Mischung 0,0001 bis 100 Gew.-%, weiter bevorzugt 0,001 bis 100 Gew.-%, besonders bevorzugt 0,1 bis 100 Gew.-%, weiter bevorzugt von 1 bis 100 Gew.-% beträgt.

D.h., erfindungsgemäße Mischungen, die nicht lediglich eine einzelne Verbindung der Formel (A) bzw. ein einzelnes Salz dieser einzelnen Verbindung der Formel (A) umfassen, können so zusammengesetzt sein, dass sie ausschließlich (100 Gew.-%) aus Verbindungen der Formel (A) und/oder deren Salzen bestehen.

Für erfindungsgemäße Salze von Verbindungen der Formel (A) gilt: Gegebenenfalls liegen eine oder mehrere Hydroxygruppen einer Verbindung der Formel (A) deprotoniert vor. Neben der bzw. den (deprotonierten) Verbindung(en) der Formel (A) liegt dabei eine entsprechende Menge an Gegenkationen vor, wobei diese vorzugsweise ausgewählt sind aus der Gruppe bestehend aus: einfach positiv geladene Kationen der ersten Hauptund Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, sowie Mischungen davon.

Die phenolischen Hydroxygruppen einer Verbindung der Formel (A) sind regelmäßig acider als Hydroxygruppen in der aliphatischen Seitenkette (sofern vorhanden).

Aus der Zahl an deprotonierten Hydroxygruppen ergibt sich die entsprechende Anzahl an Gegenkationen (in Abhängigkeit von ihrer Ladung). So ergibt sich beispielsweise für eine einem solchen Salz zugrundeliegende Verbindung der Formel (A) mit zwei phenolischen Hydroxygruppen, dass bei vollständiger Deprotonierung dieser phenolischen Hydroxygruppen ein zweifach negativ geladenes Anion vorliegt, woraus sich wiederum die Zahl an positiven Ladungen ergibt (hier: zwei), die durch das bzw. die Gegenkation(en) bereitgestellt werden muss. Besonders bevorzugt handelt es sich bei diesen Gegenkationen um Kationen ausgewählt aus der Gruppe bestehend aus Na⁺, K⁺, NH₄⁺, Ca²⁺, Mg²⁺, Al³⁺ und Zn²⁺.

Hierin beschrieben sind auch ausgewählte Verbindungen der Formel (A), die im Stand der Technik noch nicht bekannt sind, nämlich die Verbindungen Cyclohexylcannabidivarinolat (CHCBDV), Hexylcannabidivarinolat (HCBDV) und 2-Hydroxyethylcannabidivarinolat (Verbindung der Formel (IV)) (bezüglich dieser (neuen) Verbindungen der Formel (A) siehe insbesondere auch den unten folgenden Beispielteil). Entsprechendes gilt für deren Salze.

Die vorstehend genannten Verbindungen und deren Salze gehören zu den im Rahmen der vorliegenden Anmeldung besonders bevorzugt zu verwendenden Verbindungen bzw. Salzen.

Bevorzugt ist - gemäß einem Aspekt der vorliegenden Erfindung - eine erfindungsgemäße Mischung (wie vorstehend oder nachfolgend, insbesondere in den Ansprüchen definiert), wobei die Zahl der Hydroxygruppen in dem aliphatischen Rest R1 eins, zwei oder drei ist, vorzugsweise eins oder zwei. Diese Verbindungen besitzen zwar durch die Anwesenheit der besagten ein, zwei oder drei, vorzugsweise ein oder zwei Hydroxygruppen im aliphatischen Rest die Löslichkeit, welche für die vorstehend oder nachfolgend beschriebenen Anwendungen bzw. Reaktionen erforderlich ist, jedoch besitzen sie keine derart hohe Anzahl an aliphatischen Hydroxygruppen, dass unerwünschte Nebenreaktionen wie z.B. Eliminierungsreaktionen in störendem Maße auftreten.

Als besonders vorteilhaft haben sich erfindungsgemäße Mischungen erwiesen, wobei der aliphatische Rest der Verbindung der Formel (A) gesättigt und/oder unverzweigt ist, vorzugsweise gesättigt und unverzweigt ist, da ungesättigte aliphatische Reste das Risiko von unerwünschten Nebenreaktionen erhöhen und verzweigte aliphatische Reste die sterischen Anforderungen für erfindungsgemäße Mischungen in der Regel nicht in gleichem Maße erfüllen (v.a. für die Anwendung als Arzneimittel oder die Anwendung in einem Verfahren zur therapeutischen Behandlung des menschlichen oder tierischen Körpers).

In einer erfindungsgemäßen Mischung ist es bevorzugt, dass die Verbindung der Formel (A) eine Verbindung
der Formel (A-I) ist 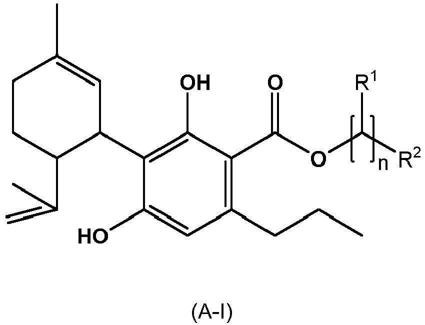 besonders bevorzugt eine Verbindung der Formel 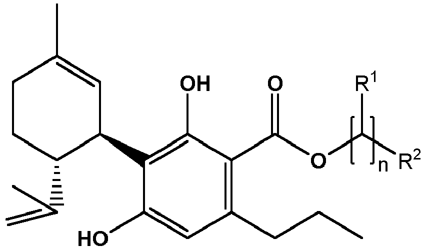 und/oder ein oder mehrere von deren Salzen (von dieser besonders bevorzugten Verbindung),
wobei gilt:
- jedes R¹: bedeutet unabhängig von der Bedeutung jedes anderen der insgesamt n Reste R¹ H, Alkyl mit ein oder zwei C-Atomen oder OH
- R²: bedeutet H oder OH
- n: bedeutet eine ganze Zahl im Bereich von 2 bis 10,
wobei zumindest einer der Reste R¹ oder der Rest R² OH bedeutet.

Besonders bevorzugt ist eine erfindungsgemäße Mischung, wobei die Verbindung der Formel (A) eine Verbindung der Formel
(A-II) ist 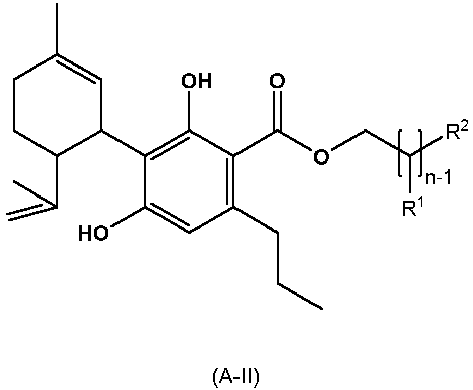 besonders bevorzugt eine Verbindung der Formel 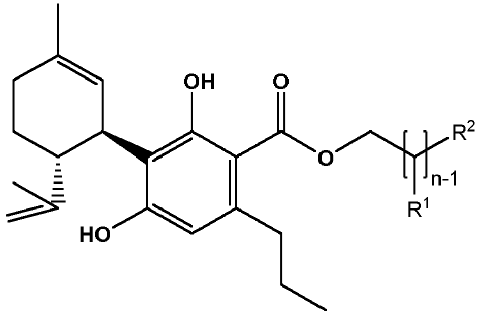 und/oder ein oder mehrere von deren Salzen (von dieser besonders bevorzugten Verbindung),
wobei gilt:
- jedes R¹: bedeutet unabhängig von der Bedeutung jedes anderen der insgesamt n-1 Reste R¹ H, Alkyl mit ein oder zwei C-Atomen oder OH
- R²: bedeutet H oder OH
- n: bedeutet eine ganze Zahl im Bereich von 2 bis 10,
wobei zumindest einer der Reste R¹ oder der Rest R² OH bedeutet.

Generell ist es bevorzugt, dass sich in Nachbarschaft zur Carboxylgruppe eine zweibindige Methylengruppe (-CH₂-) oder (sofern R¹ = H bedeutet) eine zweibindige Alkylengruppe befindet.

Ganz besonders bevorzugt ist eine erfindungsgemäße Mischung, wobei die Verbindung der Formel (A)
(i) eine Verbindung der Formel
   (A-III) ist 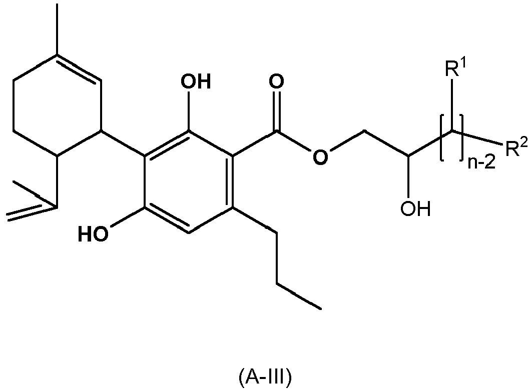 besonders bevorzugt eine Verbindung der Formel 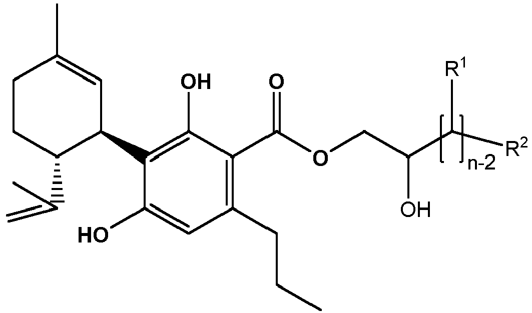 und/oder ein oder mehrere von deren Salzen (von dieser besonders bevorzugten Verbindung), wobei gilt:
   - jedes R¹: bedeutet unabhängig von der Bedeutung jedes anderen der insgesamt n-2 Reste R¹ H, Alkyl mit ein oder zwei C-Atomen oder OH
   - R²: bedeutet H oder OH
   - n: bedeutet eine ganze Zahl im Bereich von 2 bis 10, vorzugsweise im Bereich von 3 bis 10
   wobei zumindest einer der Reste R¹ oder der Rest R² OH bedeutet.
   und/oder
(ii) eine Verbindung der Formel
   (A-IV) ist 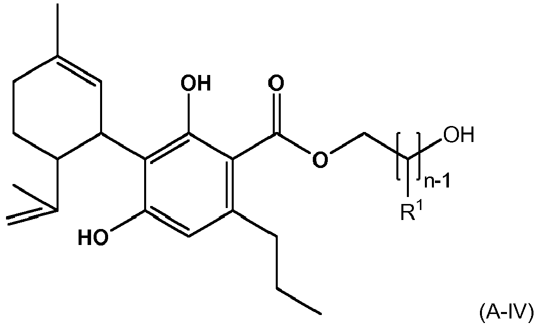 besonders bevorzugt eine Verbindung der Formel 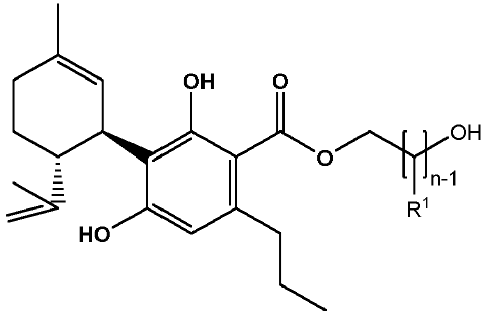
und/oder ein oder mehrere von deren Salzen (von dieser besonders bevorzugten Verbindung),
wobei gilt:
- jedes R¹: bedeutet unabhängig von der Bedeutung jedes anderen der insgesamt n-1 Reste R¹ H, Alkyl mit ein oder zwei C-Atomen oder OH
- n: bedeutet eine ganze Zahl im Bereich von 2 bis 10.

Solche Verbindungen der Formeln (A-III) und (A-IV) besitzen in der aliphatischen Seitenkette mindestens eine Hydroxygruppe und in Nachbarschaft zur Carboxylgruppe eine zweibindige Methylengruppe (-CH₂-) bzw. (sofern R¹ in Formel (A-IV) H ist) eine Alkylengruppe.

Zudem bevorzugt ist eine erfindungsgemäße Mischung (wie vorstehend oder nachfolgend, insbesondere in den Ansprüchen definiert), wobei in den besagten Formeln (A-I), (A-II), (A-III) bzw. (A-IV)
- jedes R¹: unabhängig von der Bedeutung jedes anderen der Reste R¹ H oder OH bedeutet.

Das vorstehend (und nachfolgend) zu Verbindungen der Formeln (A-I), (A-II), (A-III) bzw. (A-IV) Gesagte gilt für besonders bevorzugte Verbindungen der Formeln (A-I), (A-II), (A-III) bzw. (A-IV) entsprechend.

Es ist auch eine erfindungsgemäße Mischung bevorzugt, die ein oder mehrere Salze der Verbindungen der Formeln (A-I), (A-II), (A-III) bzw. (A-IV) (wie vorstehend und nachfolgend, insbesondere in den Ansprüchen definiert) umfasst.

Besonders bevorzugt ist eine erfindungsgemäße Mischung (wie vorstehend oder nachfolgend, insbesondere in den Ansprüchen definiert), wobei eine/die Verbindung der Formel (A) eine Verbindung der Formel (IV) ist:

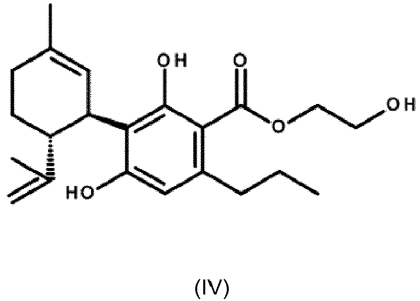

Die Erfindung betrifft auch ein Verfahren zur Herstellung einer erfindungsgemäßen Mischung (wie vorstehend oder nachfolgend, insbesondere in den Ansprüchen definiert), mit folgendem Schritt:
Umsetzen eines Cannabidiolcarbonsäureesters der Formel (IX) 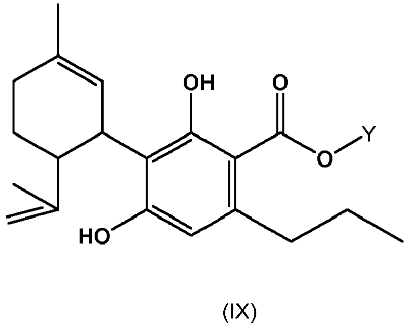 besonders bevorzugt ein Cannabidiolcarbonsäureesters der Formel 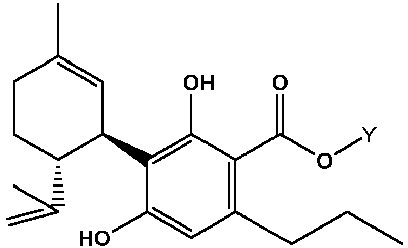 und/oder ein oder mehrere von deren Salzen (von diesem besonders bevorzugten Cannabidiolcarbonsäureester),
   wobei Y ein organischer Rest ist,
mit einem Alkohol der Formel HO-X,
wobei
X ein aliphatischer Rest mit keiner oder einer, zwei, drei oder mehr als drei Hydroxygruppen ist, wobei die Gesamtzahl der C-Atome in dem aliphatischen Rest X nicht größer ist als 15, und
wobei der aliphatische Rest
- gesättigt oder ungesättigt ist
und
- verzweigt oder unverzweigt ist,
und
- acyclisch oder cyclisch ist,
mit der Maßgabe, dass der Alkohol der Formel HO-X für den Fall, dass X ein aliphatischer Rest mit keiner Hydroxygruppe ist, ausgewählt ist aus der Gruppe bestehend aus Cyclohexanol und Hexanol,
wobei Y verschieden von X ist und so gewählt ist, dass bei der Umsetzung entstehender Alkohol der Formel HO-Y bei 1013 hPa bei niedrigerer Temperatur siedet als der eingesetzte Alkohol der Formel HO-X.

Das Produkt des erfindungsgemäßen Verfahrens ist eine erfindungsgemäße Mischung.

Bei der Umsetzung eines Esters der Formel (IX) mit Alkali in hochsiedenden Lösungsmitteln der Formel HO-X ohne Anwesenheit von Wasser wurde überraschenderweise festgestellt, dass diese Umsetzung nicht direkt zur entsprechenden Carbonsäure führt, sondern zum entsprechenden Umesterungsprodukt, d.h. einer Verbindung der Formel (A). Diese Verbindung konnte aus dem Reaktionsgemisch in hoher Ausbeute isoliert werden. Neben ihrer Eigenschaft als CB1-/CB2-Rezeptoren können diese Verbindungen der Formel (A) überdies dazu verwendet werden, CBDV und THCV herzustellen.

Besonders bevorzugt ist ein erfindungsgemäßes Verfahren zur Herstellung einer erfindungsgemäßen Mischung, wobei Y eine Alkylgruppe ist, die vorzugsweise ausgewählt ist aus der Gruppe bestehend aus Methyl, Ethyl, n-Propyl, iso-Propyl, n-Butyl, sec-Butyl, iso-Butyl, tert-Butyl.

Die Alkylgruppen Methyl, Ethyl, n-Propyl, iso-Propyl, n-Butyl, sec-Butyl, iso-Butyl und tert-Butyl haben sich als vorteilhafte Reste Y bei der Umsetzung des Esters der Formel (IX) mit einem Alkohol der Formel HO-X, herausgestellt; ihre korrespondierenden Alkohole lassen sich unter den nachfolgend beschriebenen Reaktionsbedingungen effektiv aus dem Reaktionsgemisch entfernen, was regelmäßig sowohl zu einer besonders guten Ausbeute führt als auch die Anforderungen an den Reaktionsaufbau vereinfacht.

Ganz besonders bevorzugt ist ein erfindungsgemäßes Verfahren zur Herstellung einer erfindungsgemäßen Mischung, wobei das Umsetzen des Esters der Formel (IX) mit dem Alkohol der Formel HO-X bei einem Druck erfolgt, der geringer ist als 1013 hPa, vorzugsweise bei einem Druck im Bereich von 5 bis 500 hPa.

Es ist insbesondere deshalb vorteilhaft, die Reaktion nicht bei Normaldruck, sondern unter Vakuum durchzuführen, da dies eine effiziente Entfernung des entstehenden Alkohols der Formel HO-Y (z.B. Methanol, Ethanol, n-Propanol, iso-Propanol, n-Butanol, sec-Butanol, iso-Butanol, tert-Butanol) aus dem Reaktionsgemisch ermöglicht und damit den Fortgang der Reaktion begünstigt. Der durch Umesterung entstehende Alkohol der Formel HO-Y wird vorzugsweise mittels Destillation aus dem Reaktionsgemisch entfernt.

Besonders bevorzugt ist ein erfindungsgemäßes Verfahren zur Herstellung einer erfindungsgemäßen Mischung mit folgendem Schritt zur Herstellung des Esters der Formel (IX) (das unten abgebildete Reaktionsschema zeigt die Umsetzung am (bevorzugten) Beispiel des Esters der Formel (III)):

Umsetzen von Menthadienol der Formel (I) mit einem Divarinester, besonders bevorzugt einem Divarinmethylester der Formel (II), zum entsprechenden Ester der Formel (IX), besonders bevorzugt einem Ester der Formel (III), 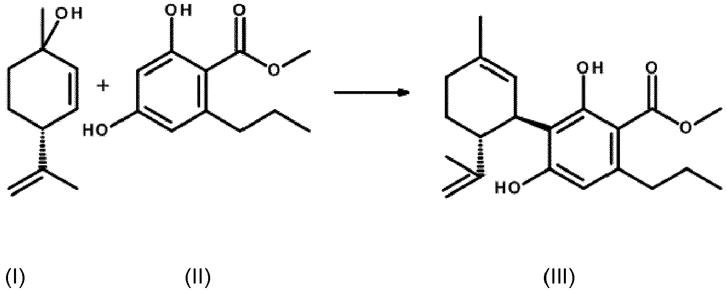 vorzugsweise in einem kontinuierlichen Verfahren.

Es wurde überraschend festgestellt, dass die Umsetzung zum Ester der Formel (IX) mit einer sehr hohen Reaktionsgeschwindigkeit abläuft, so dass das Verfahren in einer kontinuierlichen Verfahrensweise mit hoher Raum-Zeit-Ausbeute durchführbar ist. Im Rahmen der entsprechenden Untersuchung wurde eine Lösung der beiden Ausgangsprodukte mit einer Lösung eines Lewis-Säure-Katalysators kontinuierlich in eine gerührte Reaktionszelle gepumpt und anschließend in eine gesättigte wässrige Natriumbicarbonatlösung geleitet, um den Katalysator zu hydrolysieren und eine Weiterreaktion zu Nebenprodukten zu verhindern.

Die Umsetzung kann in verschiedenen Lösungsmitteln durchgeführt werden, wie z.B. Methylenchlorid, Chlorbenzol, Toluol, Xylol und Cyclohexan, wobei Methylenchlorid und Chlorbenzol deutlich bessere Ausbeuten zeigen, aus gewerbe-hygienischen Gründen jedoch hochsiedendes Chlorbenzol zu favorisieren ist.

Als Katalysatoren sind (Lewis-)Säuren wie Bortrifluorid*Etherat, Bortrifluorid*Essigsäure, Titantetrachlorid, p-Toluolsulfonsäure oder Methansulfonsäure sowie Metall-Triflate verwendbar, wobei Bortrifluorid*Etherat besonders gute Ergebnisse erzielt.

Besonders bevorzugte Ausgestaltungen ergeben sich aus dem unten folgenden Beispielteil (s. hierzu insbesondere die beschriebenen Schritte 1 "Kupplungsschritt" und 2 "Umesterung"). Wie sich ebenfalls aus dem Beispielteil ergibt, können die hierin beschriebenen Verbindungen der Formel (A) alternativ auch erhalten werden, indem der Kupplungsschritt erst nach der Umesterung erfolgt. Die vorliegende Erfindung betrifft daher auch ein Verfahren zur Herstellung einer erfindungsgemäßen Mischung bzw. einer hierin beschriebenen Verbindung der Formel (A), insbesondere der neuen Verbindungen Cyclohexylcannabidivarinolat, Hexylcannabidivarinolat und 2-Hydroxyethylcannabidivarinolat, mit folgenden Schritten:
(a) Umestern eines Divarinsäureesters, vorzugsweise eines Divarinsäuremethylesters, mit einem Alkohol der Formel HO-X,
   wobei
   X ein aliphatischer Rest mit keiner oder einer, zwei, drei oder mehr als drei Hydroxygruppen ist, wobei die Gesamtzahl der C-Atome in dem aliphatischen Rest X nicht größer ist als 15, und
   wobei der aliphatische Rest
   - gesättigt oder ungesättigt ist
   und
   - verzweigt oder unverzweigt ist,
   und
   - acyclisch oder cyclisch ist,
   mit der Maßgabe, dass der Alkohol der Formel HO-X für den Fall, dass X ein aliphatischer Rest mit keiner Hydroxygruppe ist, ausgewählt ist aus der Gruppe bestehend aus Cyclohexanol und Hexanol,
   und
(b) Umsetzen des in Schritt (a) durch Umesterung erhaltenen Divarinsäureesters mit Menthadienol zur entsprechenden Verbindung der Formel (A), vorzugsweise zu Cyclohexylcannabidivarinolat, Hexylcannabidivarinolat oder 2-Hydroxyethylcannabidivarinolat.

Die Erfindung betrifft auch ein Verfahren zur Herstellung von THCV oder von CBDV, umfassend die Herstellung einer erfindungsgemäßen Mischung bzw. einer hierin beschriebenen Verbindung der Formel (A), wobei die erfindungsgemäße Mischung vorzugsweise mittels eines erfindungsgemäßen Verfahrens (wie oben beschrieben) hergestellt wird.

Es ist besonders vorteilhaft, THCV ausgehend von einer erfindungsgemäßen Mischung bzw. einer hierin beschriebenen Verbindung der Formel (A) zu synthetisieren (vorzugsweise mittels eines erfindungsgemäßen Verfahrens (wie oben definiert) hergestellt), da die intermediär hergestellte erfindungsgemäße Mischung bzw. Verbindung sowie die üblicherweise im Anschluss intermediär gebildete Verbindung CBDV selbst individuelle biologische Aktivitäten aufweisen und somit in bestimmtem Ausmaß aus dem Verfahren abgetrennt werden können, um selbst als cannabinoide Wirksubstanzen eingesetzt zu werden. Zudem ist eine Unterbrechung des Verfahrens, eine Lagerung der intermediär hergestellten erfindungsgemäßen Mischungen und eine spätere Fortsetzung der Synthese an gleichem oder anderem Ort vorteilhafterweise möglich.

Besonders bevorzugt ist ein erfindungsgemäßes Verfahren zur Herstellung von THCV (oder von CBDV), wobei die hergestellte erfindungsgemäße Mischung bzw. die Verbindung der Formel (A) so behandelt wird, dass die (in der Mischung enthaltene) Verbindung der Formel (A) decarboxylierend verseift und die Verbindung der Formel (V) (CBDV) 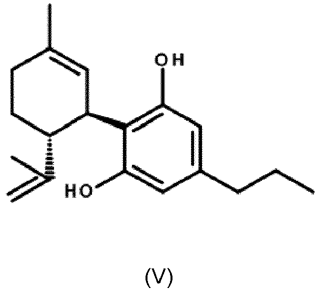 gebildet wird,
vorzugsweise wobei die nach decarboxylierender Verseifung vorliegende Verbindung (V) zu THCV cyclisiert wird, vorzugsweise in Abwesenheit halogenhaltiger Lösungsmittel.

Nachfolgend wird die vorliegende Erfindung anhand von Beispielen näher erläutert.

### Synthese von CBDV und THCV via 2-Hydroxyethylcannabidivarinolat (IV) (bevorzugute Verbindung der Formel (A) wie hierin beschrieben):

### Schritt 1: Kupplungsschritt im kontinuierlichen Verfahren): Synthese von Cannabidivarincarbonsäuremethylester (III) (bevorzugter Ester der Formel (IX) wie hierin beschrieben)

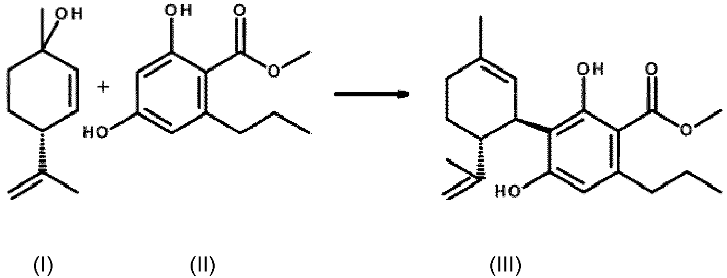

273g (1,8 Mol) Menthadienol und 377g (1,8 Mol) Divarinmethylester werden bei RT in 1.450g Toluol gelöst (2.300mL Lösung A), desgleichen werden eine adäquate Menge Bortrifluorid*Etherat in 540g Toluol bei RT gelöst (710mL Lösung B). Über zwei getrennte Dosierpumpen werden dann Lösung A mit Lösung B mit einem jeweils konstanten Fluss in eine gerührte Reaktionszelle gepumpt. Aus der Reaktorzelle läuft das Reaktionsgemisch über einen PTFE-Schlauch in eine gerührte 1.000g Natriumbicarbonat-Lösung. Die Gesamtreaktionsdauer beträgt ca. 25 min. Nach Ende der Dosierung wird die hydrolysierte Reaktionslösung noch ca. 1h nachgerührt. Man überträgt dann die hydrolysierte Reaktionslösung in einen 5-Ltr.-Doppelmantel-Reaktionsgefäß, trennt die wässrige Phase ab. Den nicht umgesetzten Divarinester extrahiert man durch sechsmalige Zugabe von 1.000g 1%-iger wässriger Natronlauge. Nach Ansäuern der vereinigten Natronlauge-Extrakte mit halbkonz. Schwefelsäure und Re-Extraktion dieser wässrigen Phase gewinnt man ca. 30% (130g) nicht umgesetzten Divarinester zurück. In der Toluolphase befinden sich ca. 320g Cannabidivarincarbonsäuremethylester (III), was einer Ausbeute von ca. 50% d.Th. entspricht. Dieses erste Zwischenprodukt dient als Ausgangsverbindung für die anschließende Umesterung.

### Alternative: Kupplungsschritt mit Metalltriflaten

Die oben beschriebene Kupplungsreaktion wird anstelle von Bortrifluorid*Etherat mit Zink-triflourmethansulfonat durchgeführt.

### Schritt 2: Umesterung / Synthese von 2-Hydroxyethylcannabidivarinolat (IV) (bevorzugte Verbindung der Formel (A) wie hierin beschrieben):

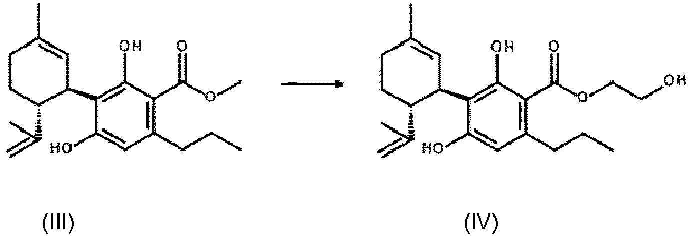

Das Toluol der aus Schritt 1 erhaltenen Lösung wird destillativ entfernt und zum verbleibenden ersten Zwischenprodukt gibt man unter Rühren 650g Ethylenglykol und versetzt mit einer Lösung aus 122g Kaliumhydroxid in 420g Ethylenglykol. Man legt ein Vakuum von ca. 0,5 bar an, und erhitzt auf 100-120°C für 2h, wobei ca. 40g Methanol abdestillieren. Das resultierende Produktgemisch umfasst hauptsächlich 2-Hydroxy-ethyl-cannabidivarinolat (IV).

### Schritt 3: Verseifung / Decarboxylierung, Synthese von CBDV (V):

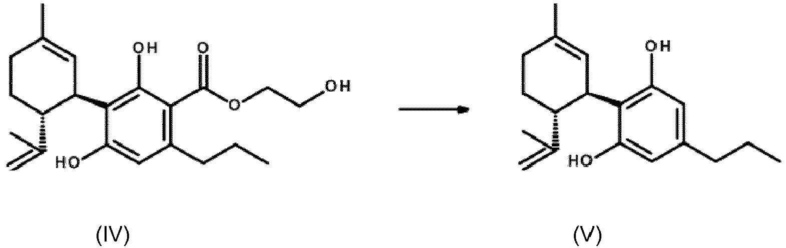

Danach erhöht man die Temperatur auf 150°C und rührt bei dieser Temperatur für 3-4h (ebenfalls unter Vakuum; vgl. Schritt 2). Man kühlt auf ca. 40°C ab und versetzt mit 1.500g Wasser sowie mit 800g Methyl-tert.-butylether und gibt zur Neutralisation ca. 180g halbkonz. Schwefelsäure hinzu. Nach Phasentrennung wird das Lösungsmittel abrotiert und der Rückstand über einen Dünnschichtverdampfer bei einem Vakuum von ca. 1 mbar und einer Manteltemperatur von 230°C destilliert. Man erhält 270g Cannabidivarin (CBDV) (V) in Form eines viskosen, gelblichen Öls mit einer Reinheit von ca. 85%; das entspricht einer Ausbeute von 85% d.Th. bezogen auf den eingesetzten Cannabidivarincarbonsäureester. Dieses viskose, gelbliche Öl wird dann in 270g n-Heptan bei ca.10°C umkristallisiert, wonach 190g weißes bis leicht gelbliches Kristallisat mit einer Reinheit von ca. 99% Cannabidivarin (V) vorliegt.

### Schritt 4: Cyclisierung, Synthese von THCV:

50g reines Cannabidivarin (V) werden in 250g Methylenchlorid und bei circa 22°C innerhalb von 10min mit 40g Bortrifluorid*Ether-Komplex unter Rühren versetzt. Man rührt 20min bei genannter Temperatur und gibt dann 200g Eiswasser hinzu, wäscht die organische Phase mit Natriumbicarbonat-Lösung und rotiert das Lösungsmittel ab. Das verbleibende Rohmaterial von ca. 50g enthält ca. 74% THCV und ca. 26% Nebenprodukte. Nach säulenchromatographischer Aufreinigung erhält man 30g reines THCV, was einer Ausbeute von ca. 60% d.Th. entspricht.

### Synthese von 2-Hydroxyethylcannabidivarinolat (IV) (bevorzugte Verbindung der Formel (A) wie hierin beschrieben) über den Divarinsäure-glykolester:

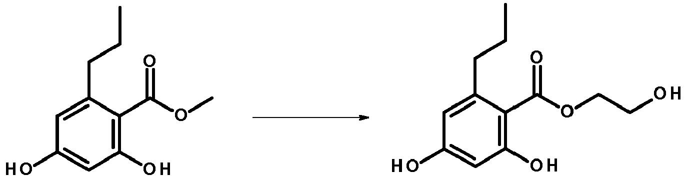

58g (0,28 Mol) Divarinsäuremethylester werden bei RT in 100g Ethylenglykol gelöst, mit einer Lösung aus 17g Natronlauge in 120g Ethylenglykol versetzt und unter Rühren 2h auf 80°C erhitzt. Man kühlt auf RT ab, gibt die Reaktionslösung auf 200g Eiswasser, gibt 200g Methyl-tert.-butylether hinzu und stellt unter Rühren mit halbkonz. Schwefelsäure auf pH 6 ein. Nach Phasentrennung entfernt man das Lösungsmittel und erhält einen bräunlich gefärbten Feststoff. Dieser wird in der gleichen Menge Toluol umkristallisiert, wonach 42g helles Kristallisat vorliegen. Ausbeute 60% d.Th.

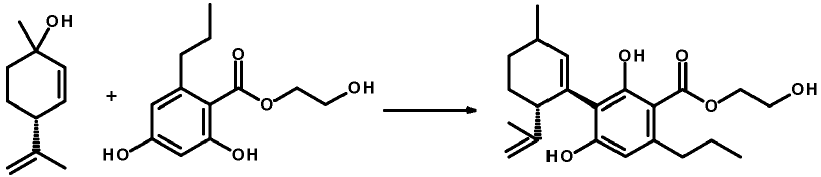

27g (0,175 Mol) Menthadienol und 42g (0,175 Mol) Divaringlykolester werden bei RT in 140g Acetonitril gelöst (250mL Lösung A), desgleichen werden eine adäquate Menge Bortrifluorid*Etherat in 63g Acetonitril bei RT gelöst (95mL Lösung B). Über zwei getrennte Dosierpumpen werden dann Lösung A mit Lösung B mit einem jeweils konstanten Fluss in eine gerührte Reaktionszelle gepumpt. Aus der Reaktorzelle läuft das Reaktionsgemisch über einen PTFE-Schlauch in eine gerührte 100g Natriumbicarbonat-Lösung. Die Gesamtreaktionsdauer beträgt ca. 5min. Nach Ende der Dosierung wird die hydrolysierte Reaktionslösung noch ca. 30min nachgerührt. Nach Zugabe von 200g trennt man die sich absetzende wässrige Phase ab und destilliert das azeotrope Gemisch aus Toluol/Acetonitril ab. Zur Abtrennung des nicht umgesetzten Divaringlykolesters versetzt man den Rückstand mit 200g frischem Toluol und extrahiert durch viermalige Zugabe von 100g 1%-iger wässriger Natronlauge. Nach Neutralisation der Toluolphase durch Waschen mit verdünnter wässriger Schwefelsäure und Abziehen des Toluols liegen 40g Rohprodukt (IV) vor. Durch säulenchromatographische Aufreinigung an Kieselgel (Cyclohexan/Essigester) lässt sich reines 2-Hydroxyethylcannabidivarinolat (IV) isolieren.

Dieses Produkt lässt sich nach o.a. Schritt 3 ins CBDV (V) überführen.

### Synthese von n-Hexylcannabidivarinolat über den Divarinsäure-n-hexylester:

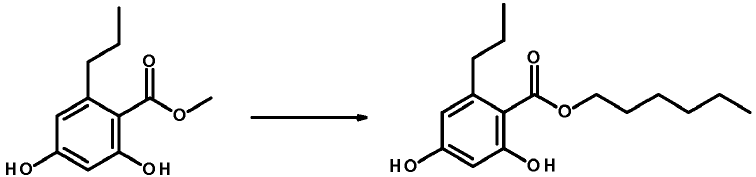

28g (0,133 Mol) Divarinsäuremethylester werden bei RT in 136g n-Hexanol gelöst, mit 0,7g (13mMol) Natriummethylat versetzt und unter Rühren 5h auf Rückfluß erhitzt. Man destilliert das Hexanol ab, gibt 100g Toluol sowie bei RT 100g Eiswasser zu, stellt unter Rühren mit halbkonz. Schwefelsäure auf pH 6. Nach Phasentrennung entfernt man das Lösungsmittel und kristallisiert in Cyclohexan um, wonach 28g weißes Kristallisat vorliegen. Ausbeute 75% d.Th.

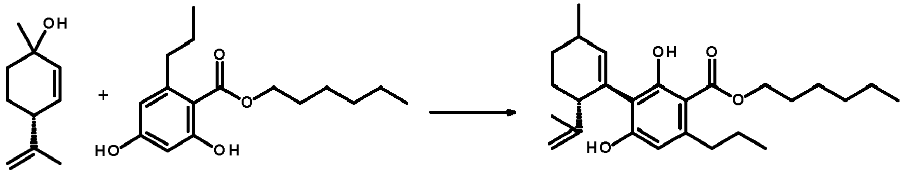

15g (0,1 Mol) Menthadienol und 28g (0,1 Mol) Divarinsäure-n-hexylester werden bei RT in 120g Toluol gelöst (150mL Lösung A), desgleichen werden eine adäquate Menge Bortrifluorid*Etherat in 25g Toluol bei RT gelöst (30mL Lösung B). Über zwei getrennte Dosierpumpen werden dann Lösung A mit Lösung B mit einem jeweils konstanten Fluss von 7mL/min in eine gerührte Reaktionszelle gepumpt. Aus der Reaktorzelle läuft das Reaktionsgemisch über einen PTFE-Schlauch in eine gerührte 100g Natriumbicarbonat-Lösung. Die weitere Aufarbeitung erfolgt analog zu dem o.a. Beispiel. Das so erhaltene rohe Produkt wird auch hier säulenchromatographisch an Kieselgel (Cyclohexan/Essigester) aufgereinigt.

Dieses Produkt lässt sich nach o.a. Schritt 3 ins CBDV (V) überführen.

### Synthese von Cyclohexylcannabidivarinolat über den Divarinsäure-cyclohexylester:

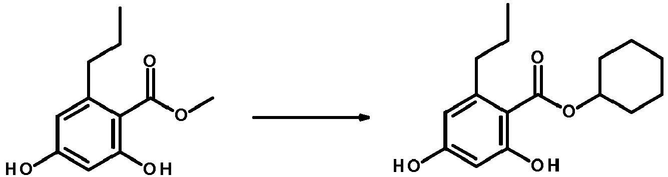

28g (0,133 Mol) Divarinsäuremethylester werden bei RT in 133g Cyclohexanol gelöst und analog zu o.a. umgesetzt. Nach Kristallisation aus Methyl-tert.-butylether erhält man 26g weißes Kristallisat. Ausbeute 70% d.Th.

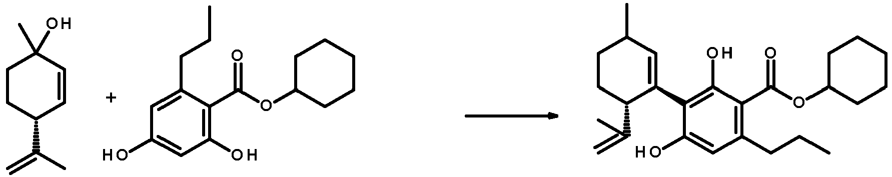

14g (94 mMol) Menthadienol und 26g (94 mmol) Divarinsäure-cyclohexylester werden bei RT in 120g Toluol gelöst (150mL Lösung A), desgleichen werden eine adequate Menge Bortrifluorid*Etherat in 25g Toluol bei RT gelöst (30mL Lösung B). Über zwei getrennte Dosierpumpen werden dann Lösung A mit Lösung B mit einem jeweils konstanten Fluss von 7mL/min in eine gerührte Reaktionszelle gepumpt. Das weitere Procedere entspricht dem o.a. Vorgehen. Das so erhaltene rohe Produkt wird ebenfalls säulenchromatographisch an Kieselgel (Cyclohexan/Essigester) aufgereinigt.

Dieses Produkt lässt sich ebenfalls nach o.a. Schritt 3 ins CBDV (V) überführen.

### Anwendungsbeispiele:

Anhand der nachfolgenden Beispiele von bevorzugten erfindungsgemäßen pharmazeutischen Formulierungen wird die erfindungsgemäße Anwendung von Verbindungen der Formel (A) näher erläutert. Bevorzugt ist insoweit der Einsatz von Verbindung (IV).

### Anwendungsbeispiel 1 - Kapseln nach dem "Neuen Rezeptur Formularium", 18. Ergänzung, 2001.

| Ansatz für 1 Kapsel | 50 mg | 100 mg | 250 mg |
|---|---|---|---|
| Verbindung der Formel (A) | 0,05 g | 0,1 g | 0,25 g |
| Hartfett (Steigschmelzp.: 37-40 °C; OH-Zahl: 7-17; VS-Zahl: 245-260) | zu 0,430 g | zu 0,430 g | zu 0,430 g |
| Hartgelatine-Steckkapselhülle, Größe 1 | 1 Stück | 1 Stück | 1 Stück |

Ansatz für 30 Kapseln einschließlich 10 % Überschuss der Schmelze

| | 50 mg | 100 mg | 250 mg |
|---|---|---|---|
| Verbindung der Formel (A) | 1,65 g | 3,3 g | 8,25 g |
| Hartfett (Steigschmelzp.: 37-40 °C; OH-Zahl: 7-17; VS-Zahl: 245-260) | zu 14,2 g | zu 14,2 g | zu 14,2 g |
| Hartgelatine-Steckkapselhüllen, Größe 1 | 30 Stück | 30 Stück | 30 Stück |

Ansatz für 60 Kapseln einschließlich 5 % Überschuss der Schmelze

| | 50 mg | 100 mg | 250 mg |
|---|---|---|---|
| Verbindung der Formel (A) | 3,15 g | 6,3 g | 16,5 g |
| Hartfett (Steigschmelzp.: 37-40 °C; OH-Zahl: 7-17; VS-Zahl: 245-260) | zu 27,1 g | zu 27,1 g | zu 27,1 g |
| Hartgelatine-Steckkapselhüllen, Größe 1 | 60 Stück | 60 Stück | 60 Stück |

1. In einer waagerecht justierten Kapselfüllmaschine werden die eingesetzten Hartgelatine-Steckkapselhüllen geöffnet, die fixierten Kapselunterteile freigelegt und zur Befüllung bereitgestellt.
2. In einem Becherglas wird etwas mehr Hartfett als für den Ansatz benötigt auf dem Wasserbad geschmolzen. Inprozessprüfung: Die Hartfettschmelze muss bei visueller Prüfung klar sein. Sie darf schwach gelb gefärbt sein.
3. In einem zweiten Becherglas wird zu der Verbindung der Formel (A) bis zur angegebenen Ansatzmenge geschmolzenes Hartfett hinzugegeben. Die Substanz wird unter Rühren mit einem Glasstab gelöst. Inprozessprüfung: Die Fettschmelze muss bei visueller Prüfung klar sein. Sie darf schwach gelb gefärbt sein.
4. Die Fettschmelze wird bis zur Befüllung der letzten Kapseln auf dem noch warmen, aber nicht mehr siedenden Wasserbad stehen gelassen, oder sie wird aus dem Wasserbad genommen und bei Bedarf wieder erwärmt. Inprozessprüfung (gelegentlich zu wiederholen): Die Temperatur der Schmelze muss zwischen 35 und 45 °C liegen.
5. Etwa 1 ml Fettschmelze wird über eine möglichst weitlumige Kanüle in eine 1-ml-Einmalspritze aufgezogen (siehe unter "Pharmazeutische Erläuterungen - Herstellungstechnik und Abfüllung"). Unverzüglich werden zwei Kapselunterteile befüllt.
   Inprozessprüfung: Der obere Rand des Kapselunterteils muss von innen vollständig mit Fettschmelze benetzt sein. Die Flüssigkeitsoberfläche muss plan oder schwach nach innen gewölbt (konkav) sein.
6. Die Spritze wird erneut befüllt, und die Füllung weiterer Kapseln wird so lange fortgesetzt, bis alle Kapselunterteile befüllt sind. Der beim Erkalten der Schmelze in den Kapseln entstehende Leerraum darf nicht weiter aufgefüllt werden. Inprozessprüfung: Im Becherglas darf nur ein kleiner Rest von etwa 1 ml Fettschmelze übrig bleiben.
7. Nach Erstarren der Fettschmelze in den Kapselunterteilen werden die Kapseln fest verschlossen.

Inprozessprüfung: Die Oberfläche der Fettschmelze muss in allen Kapselunterteilen gleichartig opak aussehen.

Endproduktprüfungen: Die verschlossenen Kapseln müssen gleichmäßig aussehen. Nur bei Bedarf: Die Einzelmassen aller Kapseln müssen zwischen 460 und 540 mg liegen. Anwendungsbeispiel 2 - Ölige Tropfen nach dem *"Neuen Rezeptur Formularium",* 19. Ergänzung, **2002.**

### Bestandteile

| | | |
|---|---|---|
| | 20 g | 100 Masseteile |
| Verbindung der Formel (A) | 5 g | 25 Teile |
| Mittelkettige Triglyceride | zu 20,0 g | zu 100,0 Teilen |

1. Die Verbindung der Formel (A) wird im Vorratsbehältnis mit einem geeigneten Solvens gelöst.
2. Die Verbindung der Formel (A) wird in ein Becherglas gewogen und unter Erwärmung und Rühren in den mittelkettigen Triglyceriden gelöst.

Endproduktprüfung: Die Lösung muss bei visueller Prüfung klar sein. Sie darf schwach gelb gefärbt sein.

## Patentansprüche

1. Mischung umfassend eine oder mehrere Verbindungen der Formel (A) und/oder ein oder mehrere von deren Salzen 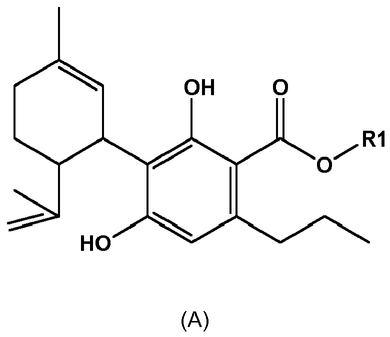 wobei R1 ein aliphatischer Rest mit keiner oder einer, zwei, drei oder mehr als drei Hydroxygruppen ist, wobei die Gesamtzahl der C-Atome in dem aliphatischen Rest R1 nicht größer ist als 15,
und wobei der aliphatische Rest
- gesättigt oder ungesättigt ist
und
- verzweigt oder unverzweigt ist
und
- acyclisch oder cyclisch ist,
mit der Maßgabe, dass die Verbindung(en) der Formel (A) bzw. das/die Salz(e) davon für den Fall, dass R1 ein aliphatischer Rest mit keiner Hydroxygruppe ist, ausgewählt ist/sind aus der Gruppe bestehend aus den Verbindungen Cyclohexylcannabidivarinolat und Hexylcannabidivarinolat sowie deren Salzen,
wobei in der Mischung
das molare Verhältnis der Gesamtmenge an Verbindungen der Formel (A) und deren Salzen zur Menge an Verbindung der Formel (V) (Cannabidivarin, CBDV) 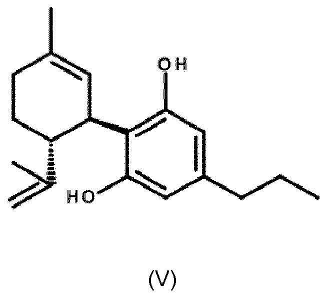 größer ist als 1 : 1, und gleichzeitig
das molare Verhältnis der Gesamtmenge an Verbindungen der Formel (A) und deren Salzen zur Menge an der Verbindung der Formel (III) ((-)-trans-Methylcannabidivarinolat) 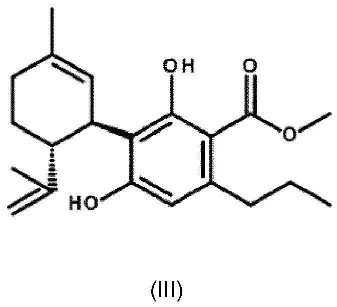 größer ist als 1 : 1.

2. Mischung nach Anspruch 1, wobei der aliphatische Rest der Verbindung der Formel (A) gesättigt und/oder unverzweigt ist, vorzugsweise gesättigt und unverzweigt.

3. Mischung nach einem der vorangehenden Ansprüche, wobei die Verbindung der Formel (A) eine Verbindung
der Formel (A-I) ist 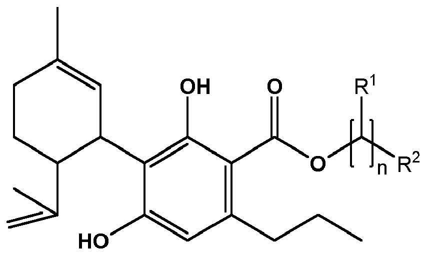 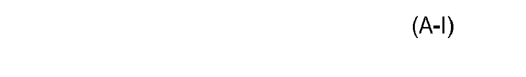 vorzugsweise eine Verbindung der Formel 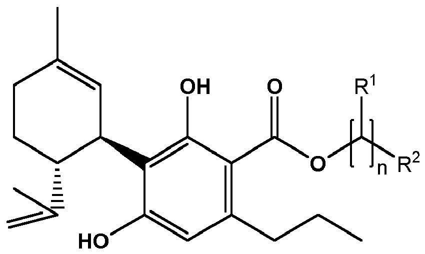 wobei gilt:
jedes R¹ bedeutet unabhängig von der Bedeutung jedes anderen der insgesamt n Reste R¹ H, Alkyl mit ein oder zwei C-Atomen oder OH
R² bedeutet H oder OH
n bedeutet eine ganze Zahl im Bereich von 2 bis 10,
wobei zumindest einer der Reste R¹ oder der Rest R² OH bedeutet,
wobei die Verbindung der Formel (A) vorzugsweise eine Verbindung der Formel
(A-II) ist 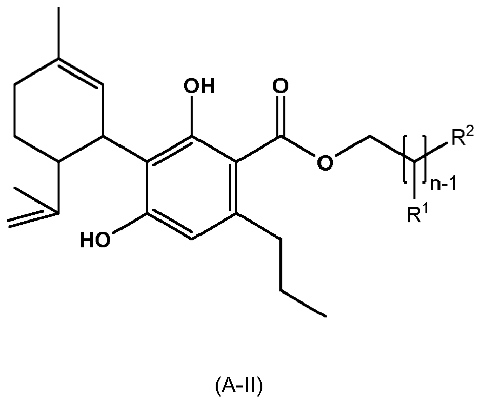 vorzugsweise eine Verbindung der Formel 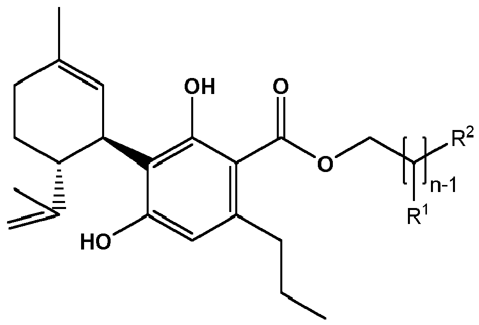 wobei gilt:
jedes R¹ bedeutet unabhängig von der Bedeutung jedes anderen der insgesamt n-1 Reste R¹ H, Alkyl mit ein oder zwei C-Atomen oder OH
R² bedeutet H oder OH
n bedeutet eine ganze Zahl im Bereich von 2 bis 10,
wobei zumindest einer der Reste R¹ oder der Rest R² OH bedeutet.

4. Mischung nach einem der vorangehenden Ansprüche, wobei die Verbindung der Formel (A)
(i) eine Verbindung der Formel
(A-III) ist 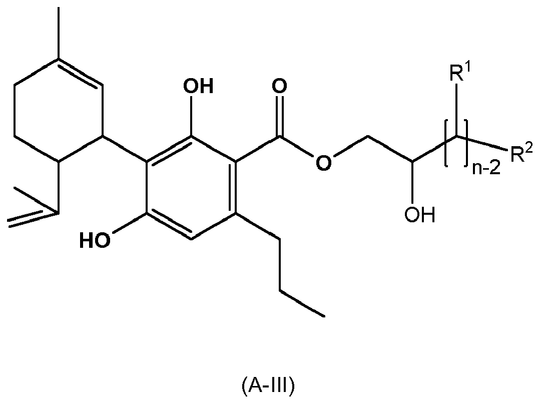 vorzugsweise eine Verbindung der Formel 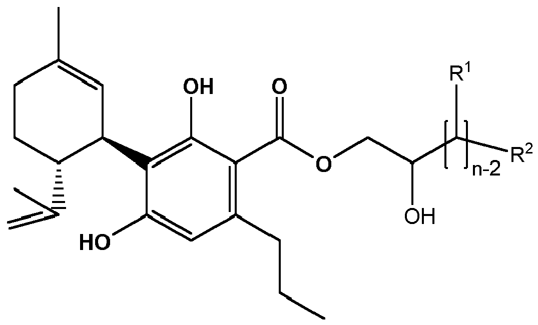 wobei gilt:
jedes R¹ bedeutet unabhängig von der Bedeutung jedes anderen der insgesamt n-2 Reste R¹ H, Alkyl mit ein oder zwei C-Atomen oder OH
R² bedeutet H oder OH
n bedeutet eine ganze Zahl im Bereich von 2 bis 10, vorzugsweise im Bereich von 3 bis 10
wobei zumindest einer der Reste R¹ oder der Rest R² OH bedeutet,
und/oder
(ii) eine Verbindung der Formel
(A-IV) ist 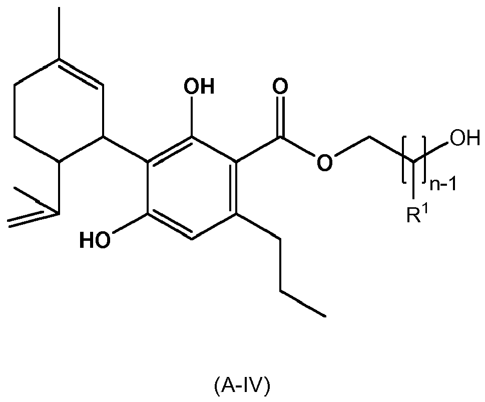 vorzugsweise eine Verbindung der Formel 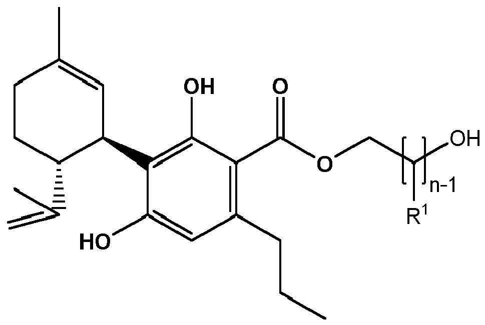 wobei gilt:
jedes R¹ bedeutet unabhängig von der Bedeutung jedes anderen der insgesamt n-1 Reste R¹ H, Alkyl mit ein oder zwei C-Atomen oder OH
n bedeutet eine ganze Zahl im Bereich von 2 bis 10.

5. Mischung nach einem der Ansprüche 3 bis 4, wobei in den besagten Formeln (A-I), (A-II), (A-III) bzw. (A-IV)
jedes R¹ unabhängig von der Bedeutung jedes anderen der Reste R¹ H oder OH bedeutet.

6. Mischung nach einem der vorangehenden Ansprüche, wobei eine/die Verbindung der Formel (A) eine Verbindung der Formel (IV) ist:

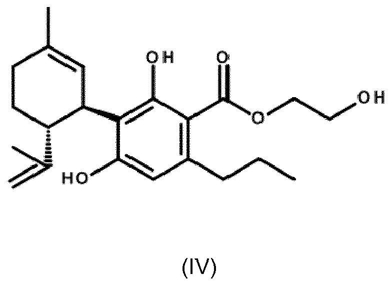

7. Verbindung der Formel (A) wie in einem der Ansprüche 1 bis 6 definiert oder Salz einer Verbindung der Formel (A) wie in einem der Ansprüche 1 bis 6 definiert oder Mischung nach einem der Ansprüche 1 bis 6
(i) zur Anwendung als Arzneimittel
oder
(ii) zur Anwendung in einem Verfahren zur therapeutischen Behandlung des menschlichen oder tierischen Körpers.

8. Verbindung der Formel (A) wie in einem der Ansprüche 1 bis 6 definiert oder Salz einer Verbindung der Formel (A) wie in einem der Ansprüche 1 bis 6 definiert oder Mischung nach einem der Ansprüche 1 bis 6, zur spezifischen Anwendung in einem Verfahren zur therapeutischen Behandlung des menschlichen oder tierischen Körpers,
zum Erreichen einer Wirkung ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
und/oder
- antiepileptische Wirkung.

9. Verfahren zur Herstellung einer Mischung gemäß einem der Ansprüche 1 bis 6 oder einer Verbindung ausgewählt aus der Gruppe bestehend aus Cyclohexylcannabidivarinolat, Hexylcannabidivarinolat und 2-Hydroxyethylcannabidivarinolat oder eines Salzes davon, mit folgendem Schritt:
Umsetzen eines Esters der Formel (IX) 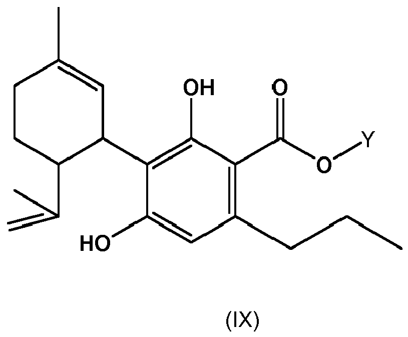 vorzugsweise eines Cannabidiolcarbonsäureesters der Formel 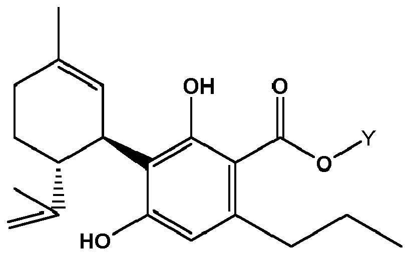 wobei Y ein organischer Rest ist,
mit einem Alkohol der Formel HO-X,
wobei
X ein aliphatischer Rest mit keiner oder einer, zwei, drei oder mehr als drei Hydroxygruppen ist, wobei die Gesamtzahl der C-Atome in dem aliphatischen Rest X nicht größer ist als 15, und
wobei der aliphatische Rest
- gesättigt oder ungesättigt ist
und
- verzweigt oder unverzweigt ist,
und
- acyclisch oder cyclisch ist,
mit der Maßgabe, dass der Alkohol der Formel HO-X für den Fall, dass X ein aliphatischer Rest mit keiner Hydroxygruppe ist, ausgewählt ist aus der Gruppe bestehend aus Cyclohexanol und Hexanol,
wobei Y verschieden von X ist und so gewählt ist, dass bei der Umsetzung entstehender Alkohol der Formel HO-Y bei 1013 hPa bei niedrigerer Temperatur siedet als der eingesetzte Alkohol der Formel HO-X.

10. Verfahren nach Anspruch 9, mit folgendem Schritt zur Herstellung des Esters der Formel (IX), bevorzugt eines Esters der Formel (III):
Umsetzen von Menthadienol der Formel (I) mit einem Divarinester, bevorzugt einem Divarinmethylester der Formel (II), zum entsprechenden Ester der Formel (IX), bevorzugt einem Ester der Formel (III), 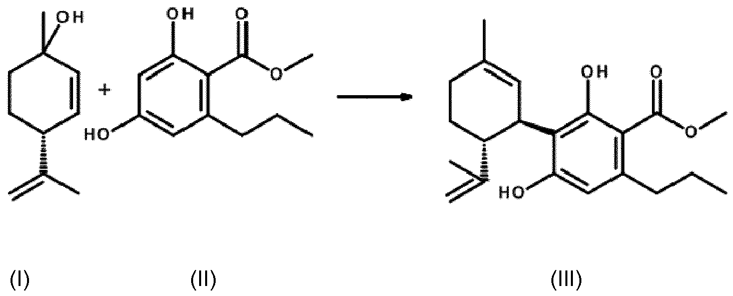
vorzugsweise in einem kontinuierlichen Verfahren.

11. Verfahren zur Herstellung einer Mischung gemäß einem der Ansprüche 1 bis 6 oder einer Verbindung ausgewählt aus der Gruppe bestehend aus Cyclohexylcannabidivarinolat, Hexylcannabidivarinolat und 2-Hydroxyethylcannabidivarinolat oder eines Salzes davon, mit folgenden Schritten:
(a) Umestern eines Divarinsäureesters, vorzugsweise eines Divarinsäuremethylesters, mit einem Alkohol der Formel HO-X,
wobei
X ein aliphatischer Rest mit keiner oder einer, zwei, drei oder mehr als drei Hydroxygruppen ist, wobei die Gesamtzahl der C-Atome in dem aliphatischen Rest X nicht größer ist als 15, und
wobei der aliphatische Rest
- gesättigt oder ungesättigt ist
und
- verzweigt oder unverzweigt ist,
und
- acyclisch oder cyclisch ist,
mit der Maßgabe, dass der Alkohol der Formel HO-X für den Fall, dass X ein aliphatischer Rest mit keiner Hydroxygruppe ist, ausgewählt ist aus der Gruppe bestehend aus Cyclohexanol und Hexanol,
und
(b) Umsetzen des in Schritt (a) durch Umesterung erhaltenen Divarinsäureesters mit Menthadienol zur entsprechenden Verbindung der Formel (A), vorzugsweise zu Cyclohexylcannabidivarinolat, Hexylcannabidivarinolat oder 2-Hydroxyethylcannabidivarinolat.

12. Verfahren zur Herstellung von THCV, umfassend die Herstellung einer Mischung gemäß einem der Ansprüche 1 bis 6 oder einer Verbindung ausgewählt aus der Gruppe bestehend aus Cyclohexylcannabidivarinolat, Hexylcannabidivarinolat und 2-Hydroxyethylcannabidivarinolat oder eines Salzes davon, vorzugsweise mittels eines Verfahrens nach einem der Ansprüche 9 bis 11.

13. Verfahren nach Anspruch 12, wobei die hergestellte Mischung gemäß einem der Ansprüche 1 bis 6 bzw. die Verbindung der Formel (A) oder das Salz davon so behandelt wird, dass die in der Mischung enthaltene Verbindung der Formel (A) bzw. dessen Salz decarboxylierend verseift und die Verbindung der Formel (V) (Cannabidivarin, CBDV) 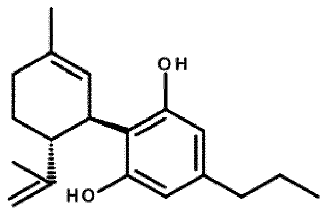 gebildet wird,
vorzugsweise wobei die nach decarboxylierender Verseifung vorliegende Verbindung (V) zu THCV cyclisiert wird, vorzugsweise in Abwesenheit halogenhaltiger Lösungsmittel.

14. Verbindung der Formel (A) wie in einem der Ansprüche 1 bis 6 definiert oder Salz einer Verbindung der Formel (A) wie in einem der Ansprüche 1 bis 6 definiert, wobei die Verbindung eine der folgenden Verbindungen ist bzw. wobei das Salz ein Salz einer der folgenden Verbindungen ist:
Cyclohexylcannabidivarinolat,
Hexylcannabidivarinolat und
2-Hydroxyethylcannabidivarinolat (Verbindung der Formel (IV)).

15. Pharmazeutische Formulierung oder kosmetische Zubereitung, umfassend eine Mischung gemäß einem der Ansprüchen 1 bis 6 oder umfassend eine oder mehrere Verbindungen gemäß Anspruch 14 und/oder ein oder mehrere Salze davon.

16. Der Ernährung und/oder dem Genuss dienende, zum Verzehr geeignete Zubereitung, umfassend eine Mischung gemäß einem der Ansprüchen 1 bis 6 oder umfassend eine oder mehrere Verbindungen gemäß Anspruch 14 und/oder ein oder mehrere Salze davon.
